# EUROPÄISCHE PATENTSCHRIFT

(11) **EP 1 557 190 B1**
(45) Veröffentlichungstag und Bekanntmachung des Hinweises auf die Patenterteilung: **15.11.2006**
(21) Anmeldenummer: 05009528.0
(22) Anmeldetag: 20.06.2001
(51) Int. Cl.: A61M 5/30, A61M 5/24, A61M 5/48, F42C 19/14

(54) **Nadellose Injektionsvorrichtung mit pyrotechnischem Antrieb**
Needleless injection device with pyrotechnic charge
Dispositif d'injection sans aiguille avec charge pyrotechnique

(30) Priorität: 20.06.2000 DE 10029325
(43) Veröffentlichungstag der Anmeldung: 27.07.2005
(62) Teilanmeldung aus: 01953796.8
(73) Patentinhaber: Lell, Peter, Dr., 85368 Moosburg (DE)
(72) Erfinder: Lell, Peter, Dr., 85368 Moosburg (DE)
(74) Vertreter: Eder, Christian

(56) Entgegenhaltungen:
- EP-A- 1 125 593
- WO-A-00/29055
- WO-A-98/31409
- US-A- 5 503 628

## Beschreibung

Die Erfindung betrifft eine nadellose Injektionsvorrichtung, mit welcher ein Wirkstoff mittels hohem Druck perkutan oder intramuskulär injiziert werden kann.

Das Grundprinzip, einen Wirkstoff nadellos mittels hohem Druck zu injizieren, ist seit geraumer Zeit bekannt. Aus der US 3,308,818 ist eine Injektionspatrone bekannt, welche ein geschlossenes Gehäuse mit einer düsenartigen Öffnung aufweist. In dem Gehäuse ist eine Treibladung vorgesehen, welche mit einer Anzündvorrichtung aktivierbar ist. Zwischen der Treibladung und der düsenartigen Öffnung ist ein sphärischer Behälter vorgesehen, in welchem der zu injizierende Wirkstoff enthalten ist. Die Injektionspatrone kann in eine revolverartige Injektionsvorrichtung eingesetzt werden, welche einen Auslösemechanismus für die Anzündvorrichtung der Injektionspatrone umfasst. Nach dem Auslösen der Anzündvorrichtung wird die Treibladung aktiviert, wobei durch den im Innenraum der Patrone entstehenden Gasdruck der Wirkstoffbehälter mit hohem Druck beaufschlagt und zusammengequetscht wird. Hierdurch platzt der Behälter im Bereich der düsenartigen Öffnung auf und der zu injizierende Wirkstoff wird mit hohem Druck aus der düsenartigen Öffnung abgegeben.

Aus der WO 98/31409 ist ein nadelloses Injektionssystem bekannt, bei dem ebenfalls in einer einmal verwendbaren Kartusche der zu injizierende Wirkstoff in einem Volumen enthalten ist, welches zumindest größtenteils von einer geschlossenen, ausreichend flexiblen und damit zusammenpressbaren Wandung umgeben ist. Das Volumen weist wiederum eine düsenartige Öffnung auf, welche im Ausgangszustand geschlossen und vor Durchführung der Injektion, beispielsweise durch das Abbrechen eines Verschlusses, geöffnet werden kann. Im rückwärtigen Bereich der Kartusche ist eine Treibladung vorgesehen, sowie eine Anzündvorrichtung zum Aktivieren der Treibladung. Durch das Aktivieren der Treibladung und den hierdurch erzeugten Gasdruck ist der in dem Volumen enthaltene Wirkstoff aus der düsenartigen Öffnung auspressbar.

Nachteilig bei derartigen Injektionspatronen oder -kartuschen ist jedoch, dass der Wirkstoff bereits von vornherein in einem abgeschlossenen Volumen der Patrone bzw. der Kartusche in einer flexiblen Hülle aufgenommen ist und damit für jede Dosierung und jeden bestimmten Wirkstoff eine entsprechende Kartusche oder Patrone hergestellt werden muss. Ein Arzt müsste demzufolge zumindest für Wirkstoffe, bei denen häufig unterschiedliche Dosierungen erforderlich sind, eine große Anzahl unterschiedlicher Kartuschen bevorraten. Des Weiteren kann es erforderlich sein, auch die Treibladung für unterschiedliche Anwendungsfälle, z.B. unterschiedliche Hauttypen, unterschiedliche gewünschte Eindringtiefen des Wirkstoffs etc., zu variieren. Hierdurch wird die Anzahl erforderlicher unterschiedlicher Kartuschen noch erhöht.

Ein weiterer Nachteil derartiger Injektionspatronen besteht darin, dass in jedem Fall sicher gestellt werden muss, dass auch über eine längere Lagerungszeit der Patronen der betreffende Wirkstoff nicht durch das Material der den Wirkstoff einschließenden flexiblen Wandung nachteilig beeinflusst wird. Da eine derartige flexible Wandung jedoch praktisch nur aus Kunststoff, beispielsweise aus PE, hergestellt werden kann, müssen entsprechend hochwertige und auch über längere Zeit hochdichte Materialien verwendet werden. Diese sind jedoch teuer. Des Weiteren muss jeder einzelne Patronentyp ein langwieriges Genehmigungsverfahren durchlaufen, da die Patrone aus solches als Medikament eingestuft wird und somit strengen Zulassungsverfahren unterliegt.

Ein weiterer Nachteil bei derartigen Injektionspatronen ist die Gefahr, dass die den Wirkstoff einschließende flexible Hülle durch den mittels der Treibladung erzeugten Gasdruck oder bei der Gaserzeugung entstehende Partikel zerstört wird. In diesem Fall würde der Wirkstoff mit dem Gas und den erzeugten Partikeln verunreinigt. Dies kann bei den Patienten zu Entzündungen oder allergischen Reaktionen führen.

Zur nadellosen Injektion sind des Weiteren mechanische Vorrichtungen bekannt, welche ein Federsystem aufweisen, das den Kolben einer Injektionskanüle mit einer ausreichend hohen Kraft beaufschlagt und den Kolben so schnell nach vorne bewegt, dass der zu injizierende Wirkstoff mit hohem Druck aus der Austrittsöffnung der Kanüle abgegeben wird. Durch die Verwendung von Einwegkanülen, in denen ein Wirkstoff in üblicher Art erst vor der durchzuführenden Injektion aufgezogen wird, unterliegen derartige Vorrichtungen und Kanülen nicht den strengen Zulassungsvorschriften für Medikamente.

Nachteilig bei rein mechanischen nadellosen Injektionssystemen ist der erforderliche hohe konstruktive Aufwand sowie ein hoher Wartungsaufwand. Zudem ist der in der Kanüle erzeugbare Druck für das Auspressen des Wirkstoffs begrenzt. Unter Umständen müssen daher relativ große Durchmesser für die düsenartige Austrittsöffnung verwendet werden, wodurch die Injektion für den Patienten schmerzhafter ist. Darüber hinaus ist bei einem nicht ausreichend hohen Druck die Eindringtiefe für den Wirkstoff nicht groß genug. Schließlich ermöglichen federgetriebene mechanische Injektionssysteme nur eine verhältnismäßig geringe Beschleunigung des Kolbens der Kanüle, so dass derartige Vorrichtungen für bestimmte Anwendungsfälle, in denen ein sehr rascher Druckanstieg gewünscht ist, nicht verwendbar sind.

Schließlich ist aus der US 5,399,163 ein nadelloses Injektionsverfahren und eine hierzu geeignete Vorrichtung bekannt, bei welcher der Kolben einer den zu injizierenden Wirkstoff enthaltenden Kanüle durch den Gasdruck einer Gaspatrone, die beispielsweise CO2-Gas enthält, beaufschlagbar ist. Diese Vorrichtung weist eine Druckverstärkereinrichtung auf, die durch Federn- und Kolbensysteme gebildet ist. Der damit verbundene konstruktive Aufwand macht ein derartiges System teuer. Zudem muss dieses System, wie alle mechanischen Systeme, häufig gewartet werden. Das System ist infolge seiner Größe und seines Gewichts zudem nur relativ umständlich zu handhaben. Wie alle mechanischen Systeme ist die erreichbare Anstiegsgeschwindigkeit des den Wirkstoff auspressenden Drucks begrenzt.

Die Kombination der Merkmale der Oberbegriffe der Ansprüche 1, 16 und 20 ist jeweils aus der US 5,503,628 bekannt.

Ausgehend von diesem Stand der Technik liegt der Erfindung die Aufgabe zu Grunde, eine nadellose Injektionsvorrichtung zu schaffen, die einfach und kostengünstig herstellbar ist, die leicht zu handhaben ist und möglichst universell einsetzbar ist.

Die Erfindung löst diese Aufgabe mit den Merkmalen der Patentansprüche 1, 16 und 20. Die Erfindung geht von der Erkenntnis aus, dass mit einem pyrotechnischen Antrieb, der zur Betätigung des Kolbens einer Kanüle dient, erforderlichenfalls ein extrem steiler Druckanstieg für das Auspressen des Wirkstoffs aus der Kanüle erzeugt werden kann. Durch die Verwendung einer Treibladungskartusche kann diese exakt auf den jeweiligen Einsatzzweck abgestimmt sein. Durch unterschiedliche Treibladungen bzw. unterschiedliche Ausbildungen und Dimensionierungen des Treibspiegels und des Kartuschenvolumens kann der jeweils gewünschte zeitliche Druckverlauf erzeugt werden. Beispielsweise ist es möglich, für die Treibladung eine Kombination aus einem schnellen lebhaften Treibladungspulver und einem langsamen, aber über einen längeren Zeitraum gaserzeugenden Treibladunspulver zu verwenden. Hierdurch kann beispielsweise ein sehr rascher Druckanstieg mit einem hohen Peak und einem sich anschließendem zunächst konstanten und dann langsam abfallenden Druckverlauf erzeugt werden. Unter einer Treibladung soll im Sinne der vorliegenden Erfindung jedes aktivierbare Gas erzeugende Material verstanden werden.

Durch das Vorsehen einer in das Gehäuse der Injektionsvorrichtung einsetzbaren Kanüle, in welche der Wirkstoff erst kurz vor der durchzuführenden Injektion eingebracht wird, ergeben sich an die Materialien der Kanüle geringere Anforderungen.

Zwischen dem zu injizierenden Wirkstoff und der Treibladung bzw. dem durch die Treibladung erzeugten Gas befinden sich sowohl der Treibspiegel als auch der Kolben der Kanüle. Da sowohl der Treibspiegel als auch der Kolben der Kanüle jeweils mit dem Umfang gegenüber dem jeweiligen Gehäuseteil auch während einer Verschiebebewegung abdichtend ausgebildet sind, ist die Gefahr einer Kontamination des Wirkstoffs durch das Treibladungsgas bzw. Partikel der Treibladung praktisch ausgeschlossen. Der Treibspiegel ist erfindungsgemäß im jeweiligen Gehäuse in seiner Ausgangsposition rastend gehalten. Des Weiteren können auch Mittel zur rastenden Halterung des Treibspiegels in einer Endposition vorgesehen sein.

Damit wird verhindert, dass der Treibspiegel bei einer Bewegung der Vorrichtung aus seiner Ausgangsposition herausbewegt wird, ohne dass der Treibspiegel mit dem Gasdruck der Treibladung beaufschlagt wird. Die rastende Halterung des Treibspiegels in der Endposition gewährleistet, dass beim Austauschen der Kartusche der Treibspiegel nicht aus der Kartusche herausfällt oder in der Injektionsvorrichtung verbleibt. Zudem bleibt bei einer derart ausgebildeten Kartusche dauernd sichtbar, dass die Kartusche bereits benutzt wurde, da der Treibspiegel nicht unabsichtlich wieder in seine Ausgangsposition bewegbar ist.

In der bevorzugten Ausführungsform kann der Treibspiegel auf seiner mit dem Gasdruck beaufschlagbaren Stirnseite im äußeren Bereich einer ringförmige Ausnehmung aufweisen, die so beschaffen ist, dass die zwischen dem Außenumfang des Treibspiegels und der ringförmigen Ausnehmung ausgebildete lidartige Wandung bei Belaufschlagung mit dem Gasdruck unter vorzugsweise elastischer Verformung gegen die Innenwandung des jeweiligen Gehäuses gepresst wird, um eine Dichtwirkung zu erzeugen. Durch diese einfache Maßnahme kann auf das Vorsehen eines O-Rings am Außenumfang des Treibspiegels verzichtet werden. Die Herstellung wird damit vereinfacht und verbilligt.

Nach einer Ausführungsform kann der Treibspiegel in einem Gehäuseteil der Injektionsvorrichtung vorgesehen sein, wobei die Treibladungskartusche als separates Teil ausgebildet und in das Gehäuseteil der Injektionsvorrichtung einsetzbar ist. Der Treibspiegel kann bei dieser Ausführungsform gegen die Rückstellkraft eines federnden Elements bewegbar sein. Hierdurch wird eine mehrfache Verwendbarkeit des Treibspiegels erreicht. Die Treibladungskartusche weist in diesem Fall keinen eigenen Treibspiegel auf, sondern ist als reine Gas erzeugende Kartusche ausgebildet. Allerdings ergeben sich bei einer derartigen Ausführungsform in dem Gehäuseteil, in dem der Treibspiegel verschiebbar gehalten ist, Schmauchspuren, so dass diese Gehäuseteil nur in begrenztem Rahmen mehrfach verwendbar ist. Das Gehäuseteil müsste häufig gereinigt und gegebenenfalls ausgetauscht werden.

Das Gehäuseteil kann in diesem Fall zusammen mit dem Treibspiegel als kartuschenartiges Austauschteil ausgebildet sein.

In einer anderen Ausführungsform ist der Treibspiegel im Gehäuse einer Kanüle vorgesehen und die Kartusche als separates Teil ausgebildet und in das Kanülengehäuse einsetzbar.

In gleicher Weise kann der Treibspiegel wiederum im Kanülengehäuse vorgesehen sein, wobei die Kartusche als separates Teil ausgebildet und an der rückwärtigen Seite der Kanüle in das Gehäuse der Injektionsvorrichtung einsetzbar sein.

Auch in diesen Fällen kann die Kartusche als reine Gas erzeugende Kartusche ohne eigenen Treibspiegel ausgebildet sein.

In einer weiteren Ausführungsform können die Kanüle und die Kartusche als ein einziges Teil ausgebildet sein, wobei der Kolben und der Treibspiegel im gemeinsamen Kanülen-/Kartuschengehäuse vorgesehen sind. Diese Ausführungsform bietet sich beispielsweise für solche Einsatzzwecke an, in denen sehr häufig dieselbe Kanülengröße mit derselben Treibladung verwendbar ist.

In allen Fällen, in denen der Treibspiegel im Kanülengehäuse oder im gemeinsamen Känüle-/Kartuschengehäuse vorgesehen ist, kann der Treibspiegel einstückig mit dem Kolben ausgebildet oder kraftschlüssig mit diesem gekoppelt sein.

Dabei kann der axiale Abstand zwischen den Dichtungsmitteln des Kolbens und den Dichtungsmitteln des Treibspiegels größer sein, als der Bewegungsweg des Treibspiegels zwischen seiner Ausgangsposition und seiner Endposition. Hierdurch ergibt sich der Vorteil, dass nicht ein und derselbe Teilbereich der Innenwandung des Gehäuses sowohl mit den Dichtungsmitteln des Treibspiegels als auch mit den Dichtungsmitteln des Kolbens zusammenwirken muss, um eine abdichtende Wirkung zu erzielen. Hierdurch ergibt sich eine verbesserte Betriebssicherheit im Hinblick auf eine Kontamination des Wirkstoffs durch das Treibgas bzw. Partikel des Treibgases.

In einer anderen Ausführungsform der Erfindung können die Kanüle und die Treibladungskartusche jeweils als separates Teil ausgebildet und in die Injektionsvorrichtung einsetzbar sein. Der Treibspiegel ist hierbei in der Treibladungskartusche vorgesehen und beaufschlagt vorzugsweise bereits nach dem Einsetzen in die Injektionsvorrichtung den Kolben der Kanüle. Bei dieser Ausführungsform wird der Treibspiegel lediglich innerhalb des Kartuschengehäuses bewegt und ist gegenüber diesem abgedichtet. Außerhalb des Kartuschengehäuses werden daher kaum Schmauchspuren erzeugt. (Eine derartige Treibladungskartusche kann auch für andere Einsatzzwecke als mechanisch wirkende pyrotechnische Antriebsvorrichtungen verwendet werden.)

Die Kanüle (ob als separates Teil oder als kombinierte Kanülen-/Kartuscheneinheit) kann nach dem Einsetzen in die Injektionsvorrichtung im Wesentlichen dem gesamten Außenumfang durch das Gehäuse der Injektionsvorrichtung abgestützt sein. Hierdurch kann die Wandstärke der Kanüle geringer ausgebildet sein, da nicht der gesamte Druck von der Kanülenwandung aufgefangen werden muss.

Erfindungsgemäß kann eine Treibladungskartusche mit oder ohne Treibspiegel oder eine Kanüle-/Treibladungskartsucheneinheit ein Co-Volumen aufweisen, welches mit dem Volumen des Gehäuses, in welchem das Gas erzeugbar ist, in der Ausgangsposition des Treibspiegels verbunden oder durch Bewegen des Treibspiegels aus seiner Ausgangsposition heraus verbindbar ist.

Durch das Vorsehen des Co-Volumens kann der Druckanstieg und auch der folgende zeitliche Verlauf der auf den Treibspiegel ausgeübten Druckkraft beeinflusst werden.

Das Co-Volumen kann als Ringraum ausgebildet sein, welcher sich um das Volumen erstreckt, in welchem das Gas erzeugbar ist.

Der Ringraum kann durch eine stirnseitige Wandung begrenzt sein, in welcher ein oder mehrere Durchbrüche vorgesehen sind, die in der Ausgangsposition durch den Treibspiegel im Wesentlichen abgedichtet sind. In der stirnseitigen Wandung ist zumindest ein weiterer, vorzugsweise zentrischer Durchbruch vorgesehen, durch welchen das durch die Treibladung erzeugte Gas den Treibspiegel beaufschlagt.

Das Co-Volumen des Ringraums kann durch ein an der rückwärtigen Stirnseite des Ringraums eingreifendes ringförmiges Element einstellbar sein. Das ringförmige Element kann beispielsweise durch einen Verstellmechanismus verschiebbar sein oder es können für unterschiedliche Anwendungszwecke unterschiedliche Ringelemente verwendet werden, die in den Ringraum eingesetzt werden.

Die Anzündvorrichtung der Treibladungskartusche oder der Kanülen-/Treibladungskartuscheneinheit ist vorzugsweise elektrisch ansteuerbar ausgebildet, wobei zwei Anschlusskontakte der Anzündvorrichtung an der rückwärtigen Stirnseite des jeweiligen Gehäuses so herausgeführt sind, dass deren Kontaktanschlussflächen innerhalb zweier gedachter, sich nicht überlappender, konzentrischer Ringbereiche oder innerhalb eines gedachten zentrischen Kreises und eines gedachten konzentrischen Ringbereichs liegen.

Die Anzündeinrichtung der nadellosen Injektionsvorrichtung für eine derartige Treibladungskartusche ohne Kanülen-/Treibladungskartsucheneinheit kann zwei ringförmige, schneidenartig ausgebildete Kontakte oder einen zentrischen Kontakt und einen ringförmigen, schneidenartig ausgebildeten Kontakt aufweisen. Durch diese Ausbildung der Anschlusskontakte der Anzündvorrichtung der Treibladungskartusche bzw. Kanülen/Treibladunskartuscheneinheit und die Ausbildung der Kontakte der Anzündeinrichtung ergibt sich eine sichere elektrische Verbindung zwischen den betreffenden Kontakten nach dem Einsetzen der Treibladungskartusche bzw. Kanülen-/Treibladungskartuscheneinheit in die Injektionsvorrichtung, ohne die Kartusche ausgerichtet einsetzen zu müssen.

Die Anzündeinrichtung umfasst vorzugsweise eine gegen die Rückstellkraft eines federnden Elements verschiebbare Batterie, wobei elektrische Verbindungsleitungen vorgesehen sind, mittels welcher die Batteriepole bei einem Verschieben der Batterie um wenigstens einen vorbestimmten Weg mit den Kontakten der Anzündeinrichtung verbindbar sind.

Die Batterie kann sich mit einem Batteriepol gegen eine elektrisch leitende flexible Membran abstützen, welche Teil der betreffenden elektrischen Verbindungsleitung ist. Die Membran ist dabei so ausgebildet, dass sie bei Beaufschlagung mit einer Druckkraft das Verschieben der Batterie um einen ausreichenden Weg ermöglicht.

Diese Ausführungsform einer Anzündeinrichtung ist mit geringem Aufwand und damit kostengünstig realisierbar. Durch den mechanisch sehr einfachen Aufbau ergibt sich eine hohe Betriebssicherheit. Sie kann, ebenso wie die spezielle Ausbildung der Kontakte der Anzündvorrichtung, auch für jede andere Vorrichtung eingesetzt werden.

Weitere Ausführungsformen der Erfindung ergeben sich aus den Unteransprüchen.

Die Erfindung wird nachfolgend anhand in der Zeichnung dargestellter Ausführungsbeispiele näher erläutert. In der Zeichnung zeigen
- Fig. 1: einen Längsschnitt durch eine Injektionsvorrichtung mit einer separaten Kanüle und einer separaten Treibladungskartusche, wobei der Treibspiegel in der Treibladungskartusche vorgesehen ist;
- Fig. 2: einen Längsschnitt durch eine Injektionsvorrichtung analog Fig. 1 mit einer in die Injektionsvorrichtung einschraubbaren Kanüle;
- Fig. 3: einen Längsschnitt durch einen Injektionsvorrichtung analog Fig. 1 mit einer mittels eines Bajonettverschlusses in die Injektionsvorrichtung einsetzbaren Kanüle;
- Fig. 4: einen Längsschnitt durch eine Injektionsvorrichtung mit einer kombinierten Kanülen-/Treibladungskartuscheneinheit;
- Fig. 5: einen Längsschnitt durch eine Injektionsvorrichtung mit einer Kanüle, in welcher ein mit dem Kolben gekoppelter Treibspiegel vorgesehen ist, und mit einer separaten Treibladungskartusche ohne Treibspiegel;
- Fig. 6: einen Längsschnitt durch eine Injektionsvorichtung mit einem mehrfach verwendbaren Gehäuseteil, in welchem ein Treibspiegel vorgesehen ist und in welches eine Treibladungskartusche einsetzbar ist.
- Fig. 7: einen Längsschnitt durch eine Injektionsvorrichtung analog Fig. 6, jedoch mit einer abgewandelten Treibladungskartusche
- Fig. 8: einen Längsschnitt durch den vorderen Bereich einer Injektionsvorrichtung mit jeweils separat ausgebildeter Kanüle und Treibladungskartusche, wobei in der Treibladungskartusche ein Co-Volumen ausgebildet ist.
- Fig. 9: einen Längsschnitt durch den vorderen Bereich einer Injektionsvorrichtung analog Fig. 8, mit einer abgewandelten Treibladungskartusche;
- Fig. 10: eine Ausführungsform einer rein Gas erzeugenden Treibladungskartusche (Fig. 10a) und ein Diagramm mit möglichen zeitlichen Verläufen des durch die Treibladungskartusche erzeugbaren Drucks (Fig. 10b);
- Fig. 11: Darstellungen analog Fig. 10, jedoch mit einer anderen Ausführungsform einer rein Gas erzeugenden Treibladungskartusche und
- Fig. 12: eine schematische Draufsicht auf die Rückseite erfindungsgemäßer Treibladungskartuschen oder Kanülen-/Treibladungskartuscheneinheiten.

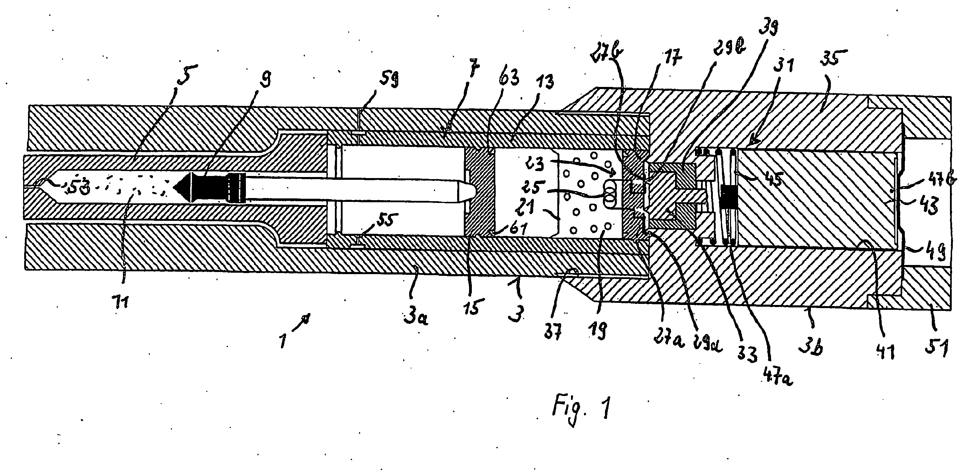

Fig. 1 zeigt eine Injektionsvorrichtung 1 mit einem Gehäuse 3, welches ein vorderes Gehäuseteil 3a und ein rückwärtiges Gehäuseteil 3b aufweist. Das vordere Gehäuseteil 3a dient zur Aufnahme einer Kanüle 5 und einer Treibladungskartusche 7. Bei der in Fig. 1 dargestellten Ausführungsform ist der Aufnahmebereich des vorderen Gehäuseteils 3a für die Kanüle 5 und die Treibladungskartusche 7 so gestaltet, dass die Kanüle und die Treibladungskartusche vom rückwärtigen Ende des Gehäuseteils einschiebbar sind.

Selbstverständlich kann das vordere Gehäuseteil 3a für das Einsetzen von Kanüle und Treibladungskartusche auch aufklappbar und nach dem Verschließen verriegelbar ausgebildet sein.

Bei der dargestellten Ausführungsform ist die Kanüle 5 im Wesentlichen ganz im Gehäuseteil 3a aufgenommen und von dessen Innenwandung abgestützt. Die Außenkontur der Kanüle 5 und die Kontur der Innenwandung des vorderen Bereichs des vorderen Gehäusebereichs 3a sind hierzu komplementär ausgebildet. Durch diese Maßnahme wird erreicht, dass die Kanüle schwächer dimensionierte Außenwandungen aufweisen kann, da der Druck, den der Kolben 9 der Kanüle 5 auf den im vorderen Bereich der Kanüle aufgenommenen Wirkstoff ausübt durch die Wandung des vorderen Gehäuseteils 3a aufgenommen wird. Trotz dünner Kanülenwandungen und eines hohen Drucks im vorderen Bereich der Kanüle können sich die Wandungen der Kanüle nicht in radialer Richtung nach außen bewegen. Die abdichtende Wirkung zwischen der Außenwandung des Kolbens und der Innenwandung der Kanüle ist somit gewährleistet.

Im rückwärtigen Bereich des vorderen Gehäuseteils 3a ist die Treibladungskartusche 7 aufgenommen, welche eine vorzugsweise zylindrische Wandung 13 in Form eines Rohrabschnitts aufweist. In der zylindrischen Wandung 13 ist ein Treibspiegel 15 vorgesehen. Im rückwärtigen Bereich der Treibladungskartusche 7 ist diese durch ein scheibenartiges Verschlusselement 17 dicht verschlossen. Sowohl die zylindrische Wandung 13 als auch der Treibspiegel 15 und das Verschlusselement 17 können aus Kunststoff bestehen. Das Verschlusselement 17 kann rastend mit der zylindrischen Wandung 13 verbunden sein. Vorzugsweise weist hierzu das Verschlusselement 17 an seinem Außenumfang eine ringförmige Erhebung auf, welche in eine ringförmige Nut in der Innenwandung der zylindrischen Wandung 13 eingreift. Selbstverständlich kann das Verschlusselement 17 auch auf andere Weise mit der Wandung 13 verbunden sein, z.B. durch Verquetschen, Verschweißen oder dergleichen.

Im rückwärtigen Bereich der Treibladungskartusche 7, vorzugsweise unmittelbar vor dem Verschlusselement 17, ist eine Treibladung 19 vorgesehen. Diese besteht vorzugsweise aus einem pyrotechnischen Material und ist mittels eines Rückhalteelement 21, das beispielsweise aus Pappe bestehen kann, im rückwärtigen Bereich der Treibladungskartusche 7 gehalten.

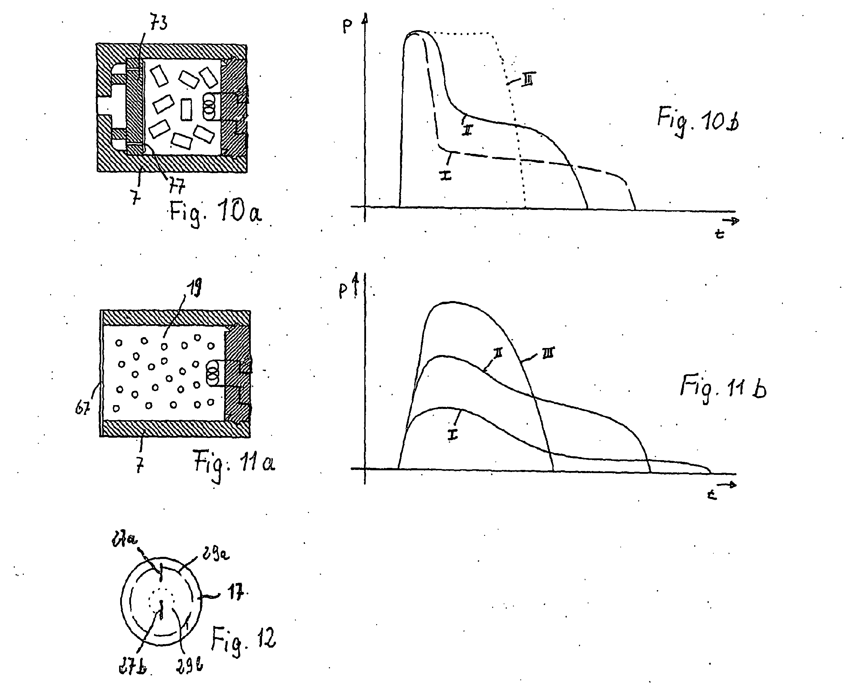

Im rückwärtigen Bereich der Treibladungskartusche ist eine Anzündvorrichtung 23 vorgesehen, die z.B. aus einer Glühwendel 25 bestehen kann, die in den Bereich mit der Treibladung 19 hineinreicht. Die Anschlusskontakte 27a, 27b der Anzündvorrichtung 23 sind an der rückwärtigen Stirnseite des Verschlusselements 17 herausgeführt. Wie in Fig. 12, welche eine schematische Draufsicht auf das Verschlusselement 17 zeigt, ersichtlich, können die Anschlusskontakte 27a, 27b der Anzündvorrichtung 23 mit ihren Kontaktanschlussflächen im Wesentlichen in radialer Richtung auf der Stirnseite des Verschlusselements 17 verlaufen. Der Anschlusskontakt 27b, der im Wesentlichen zentrisch an der Stirnseite des Verschlusselement 17 herausgeführt ist, verläuft ausgehend von seinem zentrischen Durchstoßpunkt radial nach außen. Das radial äußere Ende des Anschlusskontakts 27b endet in radialer Richtung vor dem gedachten Kreis, der durch den Durchstoßpunkt des Anschlusskontakts 27a verläuft. Auf diese Weise ist es möglich, die Anschlusskontakte 27a, 27b mittels ringförmig verlaufender Kontakte 29a, 29b einer Anzündeinrichtung 31 zu kontaktieren, die vorzugsweise als ringförmig verlaufende Schneidkontakte ausgebildet sind. In Fig. 12 sind die Linien der Schneidkontatkte 29a, 29b strichliert angedeutet.

Die Anzündeinrichtung 31, die im rückwärtigen Gehäuseteil 3b vorgesehen ist, umfasst ein erstes Kontaktelement 33, welches den ringförmigen Schneidkontakt 29b trägt. Das Kontaktelement 33 ist in einem vorderen Teil 35 des rückwärtigen Gehäuseteils 3b derart gehalten, dass nach dem Einsetzen des rückwärtigen Bereichs des vorderen Gehäuseteils 3a in eine Aufnahmeausnehmung 37 im vorderen Bereich des vorderen Teils 35 der Schneidkontakt 29b des Kontaktelements 33 einen elektrischen Kontakt mit dem Anschlusskontakt 27b der Anzündvorrichtung 23 gewährleistet ist. Das Verbinden des vorderen Teils 35 des rückwärtigen Gehäuseteils 3b und des vorderen Gehäuseteils 3a kann beispielsweise mittels einer Schraubverbindung erfolgen. Das vordere Teil 35 des rückwärtigen Gehäuseteils 3b besteht vorzugsweise aus Metall, so dass in der Aufnahmeausnehmung 37 der Kontakt 29a als ringförmige Erhebung an der bodenseitigen Wandung der Aufnahmeausnehmung 37 ausgebildet sein kann. Zur elektrischen Isolation der Kontakte 29a, 29b ist das Kontaktelement 33 mittels eines Isolierteils 39 im vorderen Teil 35 des rückwärtigen Gehäuseteils 3b gehalten. In einer Aufnahmeausnehmung 41 im rückwärtigen Bereich des Teils 35 ist eine Batterie 43 aufgenommen. Die Batterie ist in der axial verlaufenden Aufnahmeausnehmung 41 axial gegen die Kraft einer Feder 45 in Richtung auf das Kontaktelement 33 verschiebbar. Im Ausgangszustand gewährleistet die Feder 45, dass der elektrische Kontakt zwischen dem Kontaktelement 33 und dem vorderen Batteriepol 47a unterbrochen ist.

Der rückwärtige Batteriepol 47b wird von einer elektrisch leitenden flexiblen oder elastischen Membran 49 beaufschlagt, wobei die Membran 49 einen dauernden oder zumindest bei Andrücken der Membran gegen den rückwärtigen Batteriepol 47b bestehenden elektrischen Kontakt zwischen dem Batteriepol 47b und dem Teil 35 herstellt. Die Membran ist vorzugsweise mittels eines hinteren Teils 51 des rückwärtigen Gehäuseteils 3b mit dem Teil 35 verbunden, wobei die Membran 49 zwischen einander gegenüberstehenden Stirnseiten der Teile 35 und 51 eingeklemmt sein kann.

Wird auf die Membran 49 eine Druckkraft ausgeübt, die ausreicht, um die Batterie 43 gegen die Kraft der Feder 45 so weit axial zu verschieben, dass der vordere Batteriepol 47a das Kontaktelement 33 kontaktiert, so wird die Anzündvorrichtung 23 aktiviert. Im in Fig. 1 dargestellten Fall wird die Treibladung 19 mittels der Glühwendel 25, die infolge des Stromflusses erhitzt wird, angezündet.

Das erzeugte Treibgas wirkt auf den Treibspiegel 15, der den Kolben 9 beaufschlagt. Hierdurch wird der Wirkstoff 11 mit ausreichend hohem Druck in Form eines scharfen Strahls aus der düsenartigen Austrittsöffnung 53 ausgepresst.

Wie in Fig. 1 dargestellt, kann der Treibspiegel 15 in der in Fig. 1 dargestellten Ausgangsstellung mit der zylindrischen Wandung 13 der Treibladungskartusche 7 verrastet sein. Ein unbeabsichtigtes Verschieben des Treibspiegels aus seiner Ausgangsposition wird hierdurch verhindert. Das Verrasten erfolgt vorzugsweise mittels einer an der Innenwandung der Wandung 13 der Kartusche 7 ausgebildeten Erhebung, welche in eine ringförmige Nut im Außenumfang des Treibspiegels eingreift. Hierdurch ist es bei der Montage der Treibladungskartusche 7 möglich, zunächst den Treibspiegel 15 vom rückwärtigen Ende der Treibladungskartusche her einzuführen und einen derartigen Druck auf den Treibspiegel auszuüben, bis dieser in der Ausgangsstellung verrastet. Die in umgekehrter Weise ausgebildete Verrastung zwischen dem Verschlusselement 17 und der Wandung 13 ermöglicht ein einfaches Einschieben des Treibspiegels 15 bei der Montage.

In der Endposition des Treibspiegels 15 im vorderen Bereich der Treibladungskartusche 7 kann an der Innenwandung der Wandung 13 ebenfalls eine Rasterhebung ausgebildet sein. Auf diese Weise wird der Treibspiegel sicher in der Endposition fixiert und kann beim Entnehmen der Treibladungskartusche 7 aus der Injektionseinrichtung 1 nicht herausfallen oder in der Injektionsvorrichtung verbleiben. Die Kanüle dient bei der in Fig. 1 dargestellten Ausführungsform mit ihrem rückwärtigen Ende gleichzeitig als Anschlag für den Treibspiegel 15 in seiner Endposition. Selbstverständlich kann jedoch auch innerhalb der Wandung 13 der Treibladungskartusche 7 ein entsprechender Anschlag vorgesehen sein.

Die Treibladungskartusche 7 weist in ihrem vorderen Bereich eine oder mehrere Luftauslassöffnungen 55 auf, die in eine ringförmige Nut 57 im Außenumfang der Treibladungskartusche 7 münden. Im vorderen Gehäuseteil 3a sind ebenfalls eine oder mehrere Luftauslassöffnungen 59 vorgesehen, die mit der Ringnut 57 kommunizieren. Hierdurch kann die vor dem Treibspiegel befindliche Luft bei dessen Vorwärtsbewegung aus der Injektionsvorrichtung austreten, so dass ein ungewolltes Abbremsen des Treibspiegels vermieden wird.

Der Treibspiegel kann in seiner rückwärtigen Stirnseite eine umlaufende Ausnehmung 61 aufweisen, die so ausgebildet ist, dass am rückwärtigen äußeren Umfangsbereich des Treibspiegels 15 ein lidartiger ringförmiger Teilbereich 63 entsteht. Bei einer Beaufschlagung des Treibspiegels mit dem Treibladungsgas wird der lidartige Teilbereich 63 gegen die Innenwandung der Wandung 13 der Treibladungskartusche 7 gepresst, wodurch eine abdichtende Wirkung entsteht.

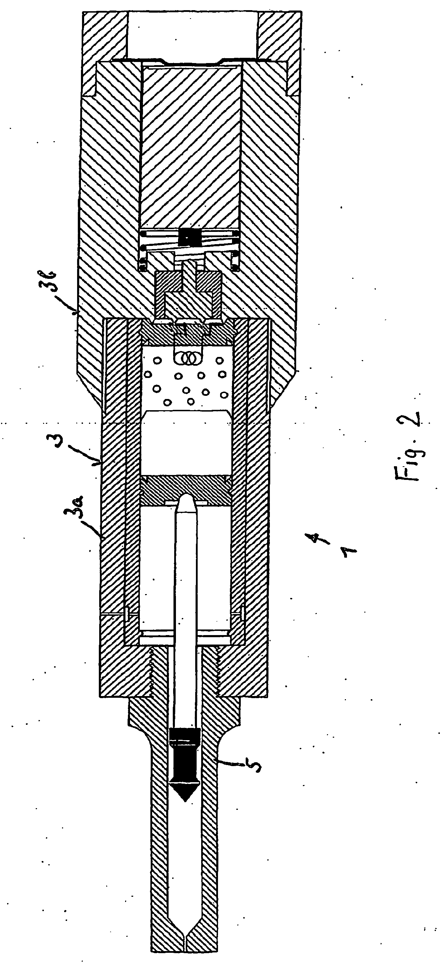

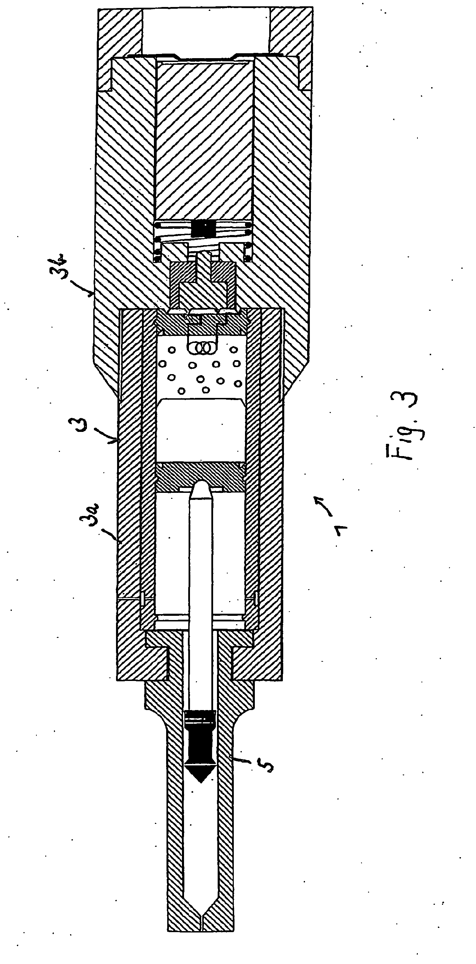

Die in Fig. 2 dargestellte Ausführungsform einer Injektionsvorrichtung 1 unterscheidet sich von der Ausführungsform nach Fig. 1 dadurch, dass die Kanüle 5 in eine Ausnehmung in der Stirnseite des vorderen Gehäuseteils 3a einschraubbar ausgebildet ist. Dementsprechend muss bei dieser Ausführungsform die Wandung der Kartusche stärker dimensioniert sein. Bei der in Fig. 3 dargestellten Ausführungsform ist die Kanüle 5 mittels eines Bajonettverschlusses mit dem Gehäuseteil 3a verbindbar. Der rückwärtige Bereich der Kanüle 5 und die Aufnahmeöffnung in der Stirnseite des Gehäuseteils 3a sind hierzu entsprechend ausgebildet. Auch bei dieser Ausführungsform muss die Wandung der Kanüle 5 stärker dimensioniert sein als bei der Ausführungsform gemäß Fig. 1.

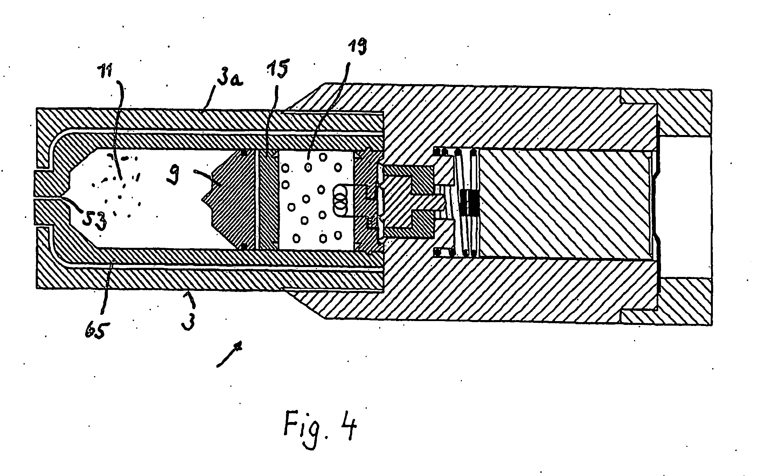

Fig. 4 zeigt eine Ausführungsform einer Injektionsvorrichtung 1, bei der die Kanüle und die Treibladungskartusche als integrierter Kanülen-/Treibladungskartuscheneinheit 65 ausgebildet sind. Im vorderen Kanülenteil der Kanülen-/Treibladungskartuscheneinheit 65 ist ein Kolben 9 vorgesehen, der zum Auspressen des vor dem Kolben befindlichen Wirkstoffs 11 aus der Austrittsöffnung 53 dient. Im rückwärtigen Treibladungskartuschenbereich der Kanülen-/Treibladungskartuscheneinheit 65 ist ein Treibspiegel 15 vorgesehen, der bei einem Anzünden der Treibladung 19 den Kolben 9 beaufschlagt und diesen nach vorne drückt.

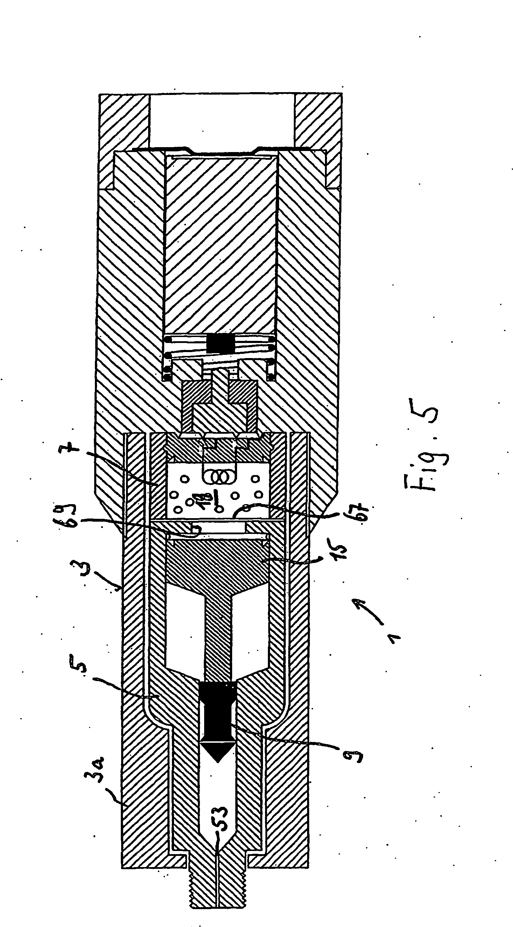

Bei der in Fig. 5 dargestellten Ausführungsform einer Injektionsvorrichtung 1 ist eine Kanüle 5 verwendet, in welcher ein Kolben 9 und ein damit starr verbundener Treibspiegel 15 vorgesehen ist. Ebenso wie bei den Ausführungsformen nach den Fig. 1 und 4 ist die Kanüle 5 im Wesentlichen ganz im Gehäuseteil 3a aufgenommen. Im rückwärtigen Bereich in unmittelbarer Nachbarschaft zur Kanüle 5 ist eine Treibladungskartusche 7 im vorderen Gehäuseteil 3a aufgenommen, welche als reine gaserzeugende Kartusche ausgebildet ist. In dieser Ausführungsform der Kartusche 7 ist kein Treibladungsspiegel enthalten. Die Treibladungskartusche 7 in Fig. 5 ist an ihrer vorderen Stirnseite mit einer Membran 67 verschlossen. Die Membran 67 hält zum Einen die Treibladung 19 im Innenraum der Treibladungskartusche 7 und zum Anderen erfüllt sie die Wirkung einer Verdämmung. Wird die Treibladung 19 angezündet, so dichtet die Membran 67 den Innenraum der Treibladungskartusche 7 so lange ab, bis der Gasdruck einen vorbestimmbaren Schwellwert überschreitet und die Membran 67 bricht oder platzt. Der Schwellendruck kann u.a. bestimmt werden durch die Dicke der Membran 67 und gegebenenfalls eine zusätzliche Abstützung der Membran.

Wie in Fig. 5 dargestellt, kann die Kanüle 5 an ihrem rückwärtigen Ende einen Durchbruch 69 aufweisen, so dass die verbleibenden Bereiche der rückwärtigen stirnseitigen Wandung der Kanüle 5 die Membran 67 abstützen. Abhängig vom Durchmesser des Durchbruchs 69 wird die Membran 67 bei einem höheren Schwellendruck (bei kleineren Durchmessern des Durchbruchs 69) oder niedrigeren Schwellendrücken (bei größeren Durchmessern des Durchbruchs 69) aufplatzen.

Die Ausbildung der Kanüle 5 gemäß Fig. 5 bietet den Vorteil, dass der Kolben und der Treibspiegel jeweils in eigenen Bereichen des Gehäuses der Kanüle 5 geführt wird. Dies verbessert die Betriebssicherheit, da bei einer Beschädigung der Innenwandung der Kanüle 5 nicht die Dichtungswirkung beider Elemente beeinträchtigt wird.

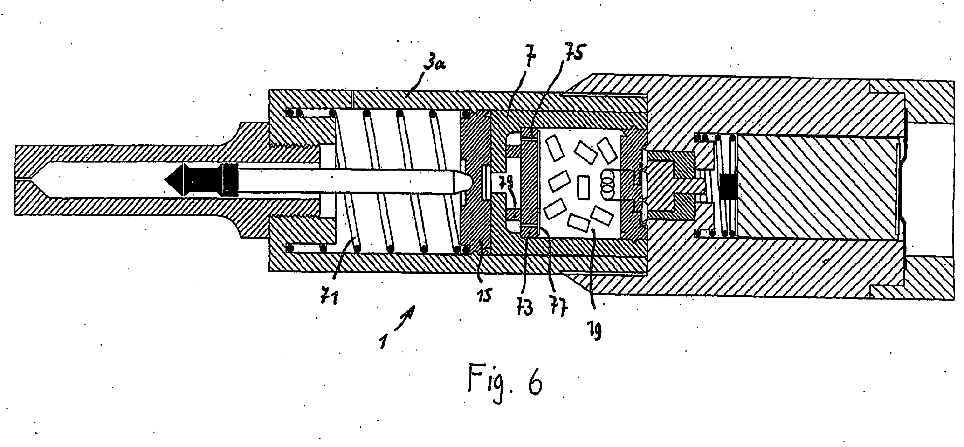

Selbstverständlich kann an Stelle einer starren Verbindung des Kolbens 9 und des Treibspiegels 15 der Kolben kraftschlüssig mit dem Treibspiegel 15 verbunden sein. Hierdurch ergibt sich der Vorteil, dass der Treibspiegel 15 in seiner Ausgangsposition verbleiben kann, und der Kolben 9 in seiner Ausgangsposition im vorderen Bereich der Kanüle vorgesehen sein kann. Erst bei einem Befüllen der Kanüle mit dem Wirkstoff durch die Austrittsöffnung 53 wird der Kolben 9 maximal so weit zurückgeschoben, bis er mit seiner Rückseite die Vorderseite des Treibspiegels 15 beaufschlagt. Die in Fig. 6 dargestellte Injektionsvorrichtung 1 umfasst ein vorderes Gehäuseteil 3a, in welchem ein Treibspiegel 15 gegen die Kraft einer Feder 71 verschiebbar ist. Im rückwärtigen Bereich des Gehäuseteils 3a ist wiederum eine rein gaserzeugende Treibladungskartusche 7 aufgenommen.

Das Gehäuseteil 3a ist bei dieser Ausführungsform einer Injektionsvorrichtung als Austauschkartusche gestaltet, da bei einem Anzünden der Treibladung 19 im Innenraum des Gehäuseteils 3a Schmauchspuren entstehen. Das Gehäuseteil 3a und der Treibspiegel 15 sind in diesem Fall zwar mehrfach verwendbar, jedoch ist ein häufiges Reinigen des Innenraums 3a erforderlich und die Kartusche in Form des Gehäuseteils 3a muss nach einer bestimmten maximalen Zahl von Verwendungen ausgetauscht werden.

Die Treibladungskartusche 7 unterscheidet sich von der ebenfalls rein gaserzeugenden Kartusche der Vorrichtung in Fig. 5 dadurch, dass im vorderen Bereich der Treibladungskartusche eine Wandung 73 vorgesehen ist, die mehrere Durchbrüche 75 geringen Querschnitts aufweist. An der Innenseite der Wandung 73 sind die Durchbrüche 75 mittels einer Membran 77 verschlossen. An ihrer Vorderseite weist die Wandung 73 mehrere Stützfüße 79 auf, die sich gegen eine stirnseitige Wandung der Treibladungskartusche 7 abstützen. Die Stützfüße 79 gewährleisten zum Einen, dass die Durchbrüche 75 nicht durch die stirnseitige Wandung der Treibladungskartusche 7 dicht verschlossen werden (in Verbindung mit der stirnseitigen Wandung der Kartusche 7), dass die Wandung 73 nicht aus der Treibladungskartusche 7 herausgedrückt wird oder bricht.

Infolge des geringen Querschnitts der Durchbrüche 75 kann eine nach dem Anzünden relativ langsam reagierende (d.h. einen langsamen Druckanstieg erzeugende) Treibladung verwendet werden. Durch die Verdämmung mittels der Wandung 73 und der Membran 77 wird erst nach Überschreiten eines sehr hohen Schwellendrucks Gas aus der Treibladungskartusche 7 abgegeben, wobei die Membran 77 bei Überschreiten dieses Schwellendrucks bricht. Die Verwendung einer langsam reagierenden Treibladung hat jedoch den Vorteil, dass der Gasdruck über längere Zeit konstant gehalten wird bzw. sich langsam abbaut, als im Fall eines schnell reagierenden Treibladungspulvers, wie dies in den Ausführungsformen nach den Fig. 1 bis 5 einsetzbar ist.

Denn bei den Ausfiihrungsformen gemäß Fig. 1 bis 5 muss zur Erzeugung eines raschen Druckanstiegs im Bereich vor dem Kolben 9 infolge des Fehlens einer wesentlichen Verdämmung eine Treibladung verwendet werden, die zumindest eine schnell reagierende Komponente umfasst. Andernfalls würde der Treibspiegel 15 und damit der Kolben 9 bei diesen Ausführungsformen infolge eines langsamen Anstiegs des Drucks des Treibladungsgases relativ langsam beschleunigt.

Selbstverständlich kann jedoch auch bei den Ausführungsformen gemäß Fig. 1 bis 5 eine langsam reagierende Treibladung verwendet werden, wenn in analoger Weise eine Verdämmung verwendet wird, wie dies in Fig. 6 mittels der Wandung 73 und der Membran 77 erfolgt.

An dieser Stelle sei darauf hingewiesen, dass bei sämtlichen Ausführungsformen auch eine Treibladung eingesetzt werden kann, die eine langsam reagierende Komponente und eine schnell reagierende Komponente umfasst. Das Mischungsverhältnis und die Art der Verdämmung (falls erforderlich) ist so zu wählen, dass sich der erforderliche Verlauf des den Treibspiegel beaufschlagenden Gasdrucks ergibt.

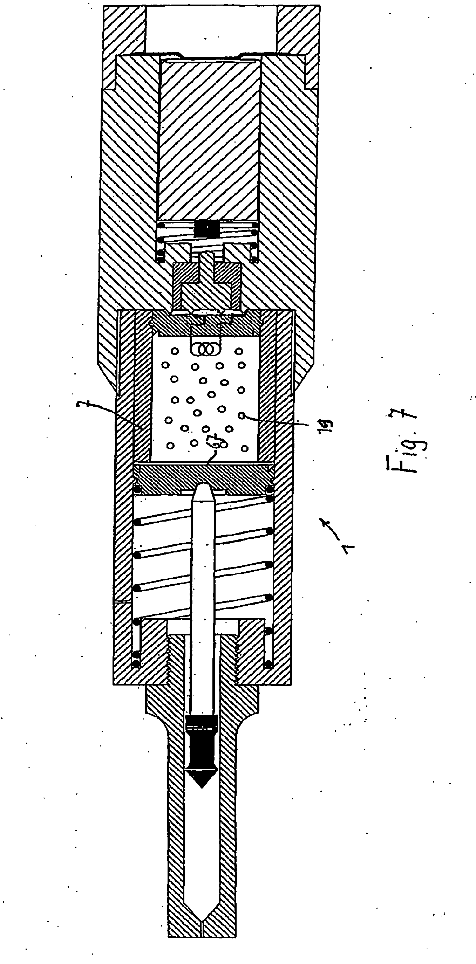

Die in Fig. 7 dargestellte Ausführungsform einer Injektionsvorrichtung 1 entspricht im Wesentlichen der Ausführungsform gemäß Fig. 6, wobei jedoch in diesem Fall wieder eine Treibladungskartusche 7 mit einer schnell reagierenden Treibladung 19 verwendet ist. Diese ist erforderlich, da die Treibladungskartusche 7 in ihrem vorderen Bereich nur mittels einer Membran 67 (analog der Membran der Treibladungskartusche in Fig. 5) verwendet ist. Eine wesentliche Verdämmungswirkung wird somit nicht erreicht.

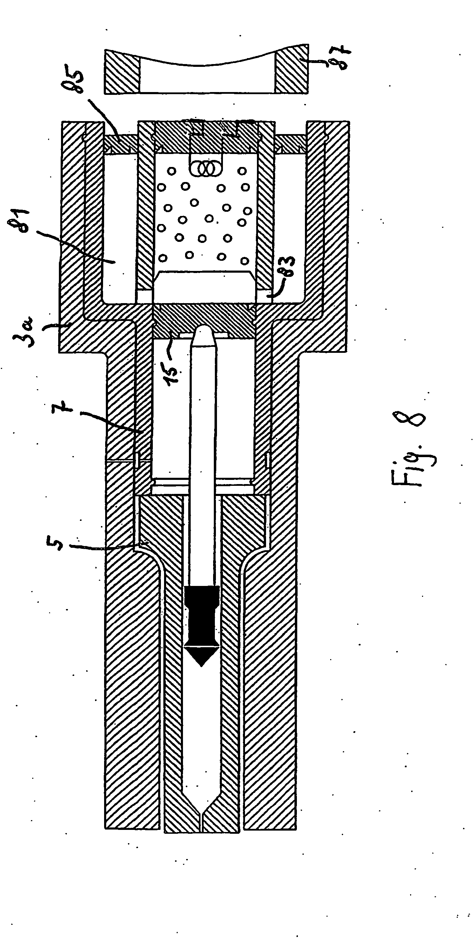

Fig. 8 zeigt eine weitere Ausführungsform einer Injektionsvorrichtung 1, die weitgehend der Ausführungsform in Fig. 1 entspricht. Die Treibladungskartusche 7 ist jedoch zusätzlich mit einem Co-Volumen 81 versehen, welches den Raum, in welchem das Treibladungsgas erzeugt wird, ringförmig umgibt. Der ringförmige Raum des Co-Volumens 81 ist über Durchbrüche 83 mit dem Raum verbunden, in welchem das Treibgas erzeugt wird. Das Co-Volumen ermöglicht die Erzeugung eines höheren Treibgasvolumens, bis der Treibspiegel 15, der ebenfalls eine geringe Dämmwirkung erzeugt, aus seiner Ausgangsposition herausbewegt wird. Bei einer Vorwärtsbewegung des Treibspiegels 15 fällt der Druck des Treibladungsgases infolge des größeren erzeugten Volumens vor einer Bewegung des Treibspiegels langsamer ab.

Das Co-Volumen 81 ist durch ein ringförmiges Dichtelement 85 abgedichtet. Das ringförmige Dichtelement 85 kann mittels eines Verstellrings 87, der in Fig. 8 nur schematisch angedeutet ist, im Ringraum des Co-Volumens 81 verschoben werden. Der Verstellring 87 kann entweder fest mit dem rückwärtigen Gehäuseteil 3b verbunden sein, welches mit dem Gehäuseteil 3a verbindbar ist, oder als eigenständiges Teil ausgebildet sein, welches zwischen dem Dichtelement 85 und dem rückwärtigen Gehäuseteil 3b eingesetzt wird. Für unterschiedliche Einsatzzwecke, die unterschiedlich große Co-Volumina 81 erfordern, können verschiedene Verstellringe 87 mit unterschiedlichen axialen Ausdehnungen verwendet werden. Selbstverständlich ist es auch möglich, im Gehäuseteil 3b einen mittels einer Verstellmechanik axial verschiebbaren Verstellring 87 vorzusehen.

Zur Abdichtung des Dichtelements 85 kann dieses, wie vorstehend in Verbindung mit dem Treibspiegel beschrieben, an der Stirnseite, die vom Gasdruck beaufschlagt wird, zwei umlaufende Ausnehmungen aufweisen, die jeweils abdichtende lidartige Bereiche definieren.

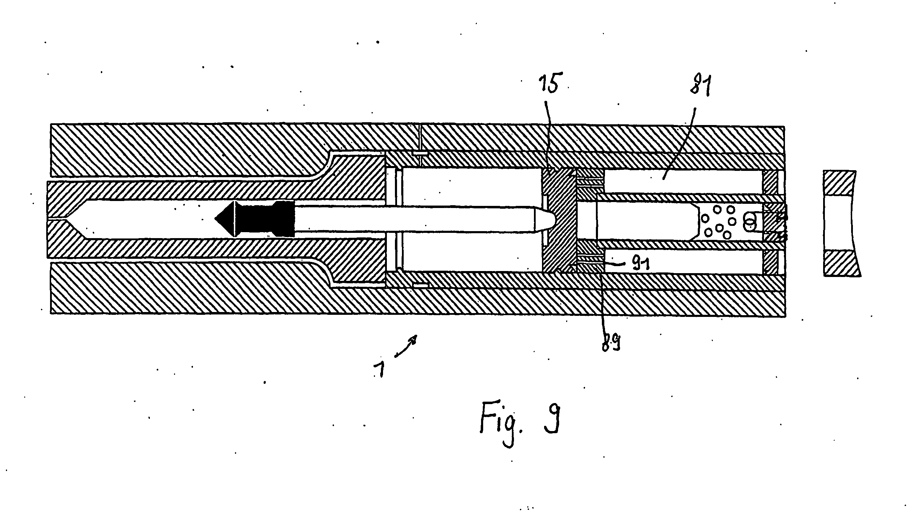

Auch die in Fig. 9 dargestellte Ausführungsform einer Injektionsvorrichtung 1 weist ein Co-Volumen 81 auf, welches sich ringförmig um den Raum erstreckt, in welchem das Treibladungsgas erzeugt wird. Zwischen dem Treibspiegel und dem ringförmigen Co-Volumen 81 ist bei dieser Ausführungsform eine Wandung 89 vorgesehen, in welcher mehrere Durchbrüche 91 vorgesehen sind.

Der Treibspiegel 15 beaufschlagt in seiner Ausgangsposition mit seiner rückseitigen Fläche die vordere stirnseitige Fläche der Wandung 89 und dichtet die Durchbrüche 81 ab. In der Ausgangsposition ist das Co-Volumen 81 daher nicht mit dem Raum verbunden, in welchem das Treibgas erzeugt wird. Erst nach dem Herausbewegen des Treibspiegels 15 aus seiner Ausgangsposition entsteht eine Verbindung zwischen dem Co-Volumen 81 und dem Volumen, in welchem das Treibgas erzeugt wird. Damit ergibt sich ein Verlauf des Gasdrucks, welcher den Treibspiegel beaufschlagt, der nach dem Herausbewegen des Treibspiegels 15 aus seiner Ausgangsposition von einem Peak auf einen geringeren Wert abfällt, diesen Wert jedoch länger aufrechterhält als dies ohne Co-Volumen der Fall wäre.

In den Fig. 10 und 11 ist nochmals dargestellt, mit welchem Treibladungskartuschentyp welcher Verlauf des Gasdrucks P realisiert werden kann, der den Treibspiegel beaufschlagt.

Fig. 10a zeigt eine rein gaserzeugende Treibladungskartusche 7, wie sie zuvor in Verbindung mit Fig. 6 beschrieben wurde. Durch die Verwendung einer relativ langsam reagierenden Treibladung (dargestellt durch grob angedeutete "Körner" oder "Tabletten" des Treibladungspulvers) und die Verdämmung mittels der Wandung 73 und der Membran 77 baut sich vor einem Durchbrechen der Membran 77 ein hoher Druck auf, der bei einem Herausbewegen des mit dem Treibgas beaufschlagten Treibspiegels aus seiner Ausgangsposition bei dem relativ geringen Kammervolumen der Treibladungskartusche 7 relativ schnell auf einen niedrigeren Wert abnimmt, wobei dieser Wert jedoch über längere Zeit gehalten werden kann, als dies mit einer ausschließlich schnell reagierenden Treibladung möglich wäre. Die verschiedenen in Fig. 10b dargestellten Verläufe des Drucks über die Zeit lassen sich mit unterschiedlichen Treibladungen realisieren. Die Kurve I kann z.B. mit einer noch relativ schnell reagierenden Treibladung erzeugt werden. Die Kurve II ist mittels einer langsamer reagierenden Treibladung realisierbar und die Kurve III mit einer sehr langsam reagierenden Treibladung oder der Kombination aus einer schnell reagierenden Treibladung und einer langsam reagierenden Treibladung.

Durch die Verdämmung der Ausführungsform der Treibladungskartusche 7 in Fig. 10a ergibt sich für alle drei Kurven I, II, III der selbe maximale Druck, bei welchem die Membran 77 durchbricht.

Bei der Ausführungsform einer rein gaserzeugenden Treibladungskartusche 7, wie sie zuvor auch in Verbindung mit der Ausführungsform einer Injektionsvorrichtung gemäß Fig. 7 beschrieben wurde, ist keine wesentlich verdämmende Wirkung gegeben. Die Membran 67 dient im Wesentlichen nur dazu, um die Treibladung 11 im Innenraum der Treibladungskartusche 7 zu halten. Dementsprechend ergibt sich bei einer langsam reagierenden Treibladung der Druckverlauf gemäß Kurve I in Fig. 11b. Die Membran 67 bricht bereits bei einem relativ geringen Maximaldruck, der dann auf einen niedrigeren Druck absinkt, der für eine relativ lange Zeit gehalten werden kann.

Bei einer schneller reagierenden Treibladung ergibt sich der Kurvenverlauf II in Fig. 11b. Die Membran 67 bricht wiederum bei einem Schwellendruck, der unterhalb des maximalen Drucks liegt. Der Maximaldruck ist im Wesentlichen nur vom Volumenstrom der Gaserzeugung abhängig. Dementsprechend ergibt sich ein höherer maximaler Druck als bei Kurve I, ein kürzerer zeitlicher Bereich, in dem der Druck annähernd konstant gehalten werden kann bzw. leicht abfällt und ein weiterer Bereich, in dem der Druck rasch auf Null abfällt.

Bei einer schnell reagierenden Treibladung ergibt sich ein sehr hoher maximaler Druck, praktisch kein Bereich, in dem der Druck über längere Zeit annähernd konstant gehalten werden kann, so dass die Kurve III relativ rasch vom Maximaldruck auf Null abfällt.

Abschließend sei darauf hingewiesen, dass selbstverständlich in Verbindung mit bestimmten Ausfiihrungsformen beschriebene Merkmale der Injektionsvorrichtung, der Kanüle, der Treibladungskartusche und der Anzündeinrichtung auch analog auf andere Ausführungsformen übertragbar sind. Die dargestellten und/oder beschriebenen Ausfiihrungsbeispiele der Treibladungskartuschen können gegebenenfalls auch für andere Verwendungszwecke eingesetzt werden, als für eine nadellose Injektionsvorrichtung.

Wichtige Aspekte der vorliegenden Erfindung können durch die folgenden Angaben zusammengefaßt werden, die sich auf spezifische und vorteilhafte Merkmale der vorliegenden Erfindung beziehen.
1. Nadellose Injektionsvorrichtung
   a) mit einer in ein Gehäuse (3) einsetzbaren Kanüle (5), welche mit einem vorbestimmten zu injizierenden Wirkstoff (11) befüllbar ist und welche einen in einem Kanülengehäuse verschiebbaren Kolben (9) für das Auspressen des Wirkstoffs (11) aus einer im Kanülengehäuse vorgesehenen Austrittsöffnung (53) aufweist,
   b) mit einer in das Gehäuse (3) einsetzbaren Treibladungskartusche (7), welche ein Kartuschengehäuse mit einer darin vorgesehenen Treibladung (19) und eine Anzündvorrichtung (23) für das Anzünden der Treibladung umfasst, und
   c) mit einem in einem Gehäuseteil (3a) der Injektionsvorrichtung (1) oder im Kanülengehäuse der Kanüle (5) oder im Kartuschengehäuse verschiebbaren Treibspiegel (15), welcher nach dem Anzünden der Treibladung (19) durch den entstehenden Gasdruck aus einer Ausgangsposition in eine Endposition bewegbar ist und dessen Außenumfang gegenüber dem Innenumfang des jeweiligen Gehäuses auch während der gesamten Verschiebebewegung abdichtend ausgebildet ist, wobei der Treibspiegel (15) den im Kanülengehäuse verschiebbaren Kolben (9) beaufschlagt.
2. Injektionsvorrichtung nach Aspekt 1, wobei der Treibspiegel (15) im jeweiligen Gehäuse in seiner Ausgangsposition rastend gehalten ist.
3. Injektionsvorrichtung nach Aspekt 1 oder 2, wobei im jeweiligen Gehäuse Mittel zur rastenden Halterung des Treibspiegels (15) in seiner Endposition vorgesehen sind.
4. Injektionsvorrichtung nach einem der vorhergehenden Aspekte, wobei der Treibspiegel (15) auf seiner mit dem Gasdruck beaufschlagbaren Stirnseite im äußeren Bereich eine ringförmige Ausnehmung aufweist, die so beschaffen ist, dass die zwischen dem Außenumfang des Treibspiegels (15) und der ringförmigen Ausnehmung ausgebildete lidartige Wandung (63) bei Beaufschlagung mit dem Gasdruck unter vorzugsweise elastischer Verformung gegen die Innenwandung des jeweiligen Gehäuses gepresst wird, um eine Dichtwirkung zu erzeugen.
5. Injektionsvorrichtung nach einem der vorhergehenden Aspekte, wobei der Treibspiegel (15) in einem Gehäuseteil (3a) der Injektionsvorrichtung (1) vorgesehen ist und dass die Treibladungskartusche (7) als separates Teil ausgebildet und in das Gehäuseteil (3a) der Injektionsvorrichtung (1) einsetzbar ist.
6. Injektionsvorrichtung nach Aspekt 5, wobei der Treibspiegel (15) vom Gasdruck gegen die Rückstellkraft eines federnden Elements (71) bewegbar ist.
7. Injektionsvorrichtung nach Aspekt 5 oder 6, wobei das Gehäuseteil (3a) als kartuschenartiges Austauschteil ausgebildet ist.
8. Injektionsvorrichtung nach einem der Aspekte 1 bis 4, wobei der Treibspiegel (15) im Kanülengehäuse vorgesehen ist und dass die Treibladungskartusche (7) als separates Teil ausgebildet und in das Kanülengehäuse einsetzbar ist.
9. Injektionsvorrichtung nach einem der Aspekte 1 bis 4, wobei der Treibspiegel (15) im Kanülengehäuse vorgesehen ist und dass die Treibladungskartusche (7) als separates Teil ausgebildet und an der rückwärtigen Seite der Kanüle (5) in das Gehäuse (3) der Injektionsvorrichtung (1) einsetzbar ist.
10. Injektionsvorrichtung nach einem der Aspekte 1 bis 4, wobei die Kanüle (5) und die Treibladungskartusche (7) als Kanülen-/Treibladungskartuscheneinheit (65) ausgebildet sind, wobei der Kolben (9) und der Treibspiegel (15) im gemeinsamen Kanülen-/Kartuschengehäuse vorgesehen sind.
11. Injektionsvorrichtung nach einem der Aspekte 8 bis 10, wobei der Treibspiegel (15) einstückig mit dem Kolben (9) ausgebildet oder kraftschlüssig mit diesem gekoppelt ist.
12. Injektionsvorrichtung nach Aspekt 11, wobei der axiale Abstand zwischen den Dichtungsmitteln des Kolbens (9) und den Dichtungsmitteln des Treibspiegels (15) größer ist als der Bewegungsweg des Treibspiegels (15) zwischen seiner Ausgangsposition und seiner Endposition.
13. Injektionsvorrichtung nach einem der Aspekte 1 bis 4, wobei die Kanüle (5) und die Treibladungskartusche (7) als separate Teile ausgebildet sind, wobei der Treibspiegel (15) im Kartuschengehäuse vorgesehen ist und vorzugsweise nach dem Einsetzen von Kanüle (5) und Treibladungskartusche (7) in die Injektionsvorrichtung (1) mit dem Kolben (9) kraftschlüssig oder formschlüssig gekoppelt ist.
14. Injektionsvorrichtung nach einem der vorhergehenden Aspekte, wobei die Kanüle (5) mit einem rückwärtigen Bereich in das Gehäuse der Injektionsvorrichtung (1) oder das Gehäuseteil (3a) der Injektionsvorrichtung, in welchem der Treibspiegel (15) vorgesehen ist, einschraubbar oder mittels eines Bajonettverschlusses einsetzbar ist.
15. Injektionsvorrichtung nach einem der vorhergehenden Aspekte, wobei die Kanüle (5) an im Wesentlichen dem gesamten Außenumfang durch das Gehäuse (3) der Injektionsvorrichtung (1) abgestützt ist.
16. Injektionsvorrichtung nach Aspekt 15, wobei die Kanüle (5) vorzugsweise in ihrem rückwärtigen Bereich einen Anlagefläche aufweist, die mit einer im Wesentlichen komplementär ausgebildeten Abstützfläche des Gehäuses (3) die Kanüle (5) bei einer Beaufschlagung des Kolbens (9) der Kanüle (5) zum Auspressen des Wirkstoffs (11) für das Abstützen der Kanüle (5) in axialer Richtung zusammenwirkt.
17. Treibladungskartusche für eine nadellose Injektionsvorrichtung, insbesondere nach einem der vorhergehenden Aspekte,
   a) mit einem Kartuschengehäuse, einer darin vorgesehenen aktivierbaren Treibladung (19) und einer Anzündvorrichtung (23) für das Aktivieren der Treibladung und
   b) mit einem im Kartuschengehäuse angeordneten, gegenüber der Gehäuseinnenwandung abdichtenden Treibspiegel (15),
   c) welcher durch den nach dem Anzünden der Treibladung (19) entstehenden Gasdruck zum Antreiben des Kolbens (9) der den zu injizierenden Wirkstoff enthaltenden Kanüle (5)aus einer Ausgangsposition in eine Endposition bewegbar ist und dessen Außenumfang gegenüber dem Innenumfang des Kartuschengehäuses auch während der gesamten Verschiebebewegung abdichtend ausgebildet ist.
18. Treibladungskartusche nach Aspekt 17, wobei der Treibspiegel (15) im Kartuschengehäuse in seiner Ausgangsposition rastend gehalten ist, wobei vorzugsweise an der Innenwandung des Kartuschengehäuses Rastvorsprünge vorgesehen sind, welche mit den Umfangskanten des Treibspiegels (15) oder einer oder mehrere in seiner Umfangsfläche vorgesehenen Rastausnehmungen zusammenwirken.
19. Treibladungskartusche nach Aspekt 17 oder 18, wobei im Kartuschengehäuse Mittel zur rastenden Halterung des Treibspiegels (15) in seiner Endposition vorgesehen sind, wobei vorzugsweise an der Innenwandung des Kartuschengehäuses entsprechende Rastvorsprünge vorgesehen sind, welche mit den Umfangskanten des Treibspiegels (15) oder einer oder mehrere in seiner Umfangsfläche vorgesehenen Rastausnehmungen zusammenwirken.
20. Treibladungskartusche nach einem der Aspekte 17 bis 19, wobei der Treibspiegel (15) auf seiner mit dem Gasdruck beaufschlagbaren Stirnseite im äußeren Bereich eine ringförmige Ausnehmung aufweist, die so beschaffen ist, dass die zwischen dem Außenumfang des Treibspiegels und der ringförmigen Ausnehmung ausgebildete lidartige Wandung (63) bei Beaufschla-gung mit dem Gasdruck unter vorzugsweise elastischer Verformung gegen die Innenwandung des Kartuschengehäuses gepresst wird, um eine Dichtwirkung zu erzeugen.
21. Kanülen-/Treibladungskartuscheneinheit für eine Vorrichtung zur nadellosen Injektion, insbesondere nach einem der Aspekte 1 bis 16,
   a) mit einem Kanülen-/Kartuschengehäuse, einer darin vorgesehenen aktivierbaren Treibladung (19) und einer Anzündvorrichtung (23) für das Aktivieren der Treibladung,
   b) mit einem im Kanülen-/Kartuschengehäuse abdichtend verschiebbaren Kolben (9) für das Auspressen des Wirkstoffs (11) aus einer im Kanülengehäuse vorgesehenen Austrittsöffnung (53) und
   c) mit einem im Kanülen-/Kartuschengehäuse angeordneten, gegenüber der Gehäuseinnenwandung abdichtenden Treibspiegel (15), welcher durch den nach dem Anzünden der Treibladung (19) entstehenden Gasdruck zum Antreiben des Kolbens (9) aus einer Ausgangsposition in eine Endposition bewegbar ist und dessen Außenumfang gegenüber dem Innenumfang des Kanülen-/Kartuschengehäuses auch während der gesamten Verschiebebewegung abdichtend ausgebildet ist.
22. Kanülen-/Treibladungskartuscheneinheit nach Aspekt 21, wobei der Treibspiegel (15) im Kanülen-/Kartuschengehäuse in seiner Ausgangsposition rastend gehalten ist, wobei vorzugsweise an der Innenwandung des Kanülen-/Kartuschengehäuses Rastvorsprünge vorgesehen sind, welche mit den Umfangskanten des Treibspiegels (15) oder einer oder mehrere in seiner Umfangsfläche vorgesehenen Rastausnehmungen zusammenwirken.
23. Kanülen-/Treibladungskartuscheneinheit nach Aspekt 21 oder 22, wobei im Kanülen-/Kartuschengehäuse ein Anschlag vorgesehen ist, der die Endposition des Treibspiegels (15) definiert.
24. Kanülen-/Treibladungskartuscheneinheit nach einem der Aspekte 21 bis 23, wobei der Treibspiegel (15) auf seiner mit dem Gasdruck beaufschlagbaren Stirnseite im äußeren Bereich eine ringförmige Ausnehmung aufweist, die so beschaffen ist, dass die zwischen dem Außenumfang des Treibspiegels und der ringförmigen Ausnehmung ausgebildete lidartige Wandung (63) bei Beaufschlagung mit dem Gasdruck unter vorzugsweise elastischer Verformung gegen die Innenwandung des Kanülen-/Kartuschengehäuses gepresst wird, um eine Dichtwirkung zu erzeugen.
25. Kanülen-/Treibladungskartuscheneinheit nach einem der Aspekte 21 bis 24, wobei der Treibspiegel (1 5) einstückig mit dem Kolben (9) ausgebildet oder kraftschlüssig mit diesem gekoppelt ist.
26. Kanülen-/Treibladungskartuscheneinheit nach Aspekt 25, wobei der axiale Abstand zwischen den Dichtungsmitteln des Kolbens (9) und den Dichtungsmitteln des Treibspiegels (15) größer ist als der Bewegungsweg des Treibspiegels (15) zwischen seiner Ausgangsposition und seiner Endposition.
27. Treibladungskartusche nach einem der Aspekte 17 bis 20 oder Kanülen/Treibladungskartuscheneinheit nach einem der Aspekte 21 bis 26, wobei das jeweilige Gehäuse ein Co-Volumen (81) aufweist, welches mit dem Volumen des Gehäuses, in welchem das Gas erzeugbar ist, in der Ausgangsposition des Treibspiegels (15) verbunden oder durch das Bewegen des Treibspiegels (15) aus der Ausgangsposition heraus verbindbar ist.
28. Treibladungskartusche oder Kanülen-/Treibladungskartuscheneinheit nach Aspekt 27, wobei, das Co-Volumen (81) als Ringraum ausgebildet ist, der sich um das Volumen erstreckt, in welchem das Gas erzeugbar ist.
29. Treibladungskartusche oder Kanülen-/Treibladungskartuscheneinheit nach Aspekt 28, wobei der Ringraum durch eine stirnseitige Wandung (89) begrenzt ist, in welcher ein oder mehrere Durchbrüche (91) vorgesehen sind, die in der Ausgangsposition durch den Treibspiegel (15) im Wesentlichen abgedichtet sind, dass in der stirnseitigen Wandung (89) wenigstens ein Durchbruch vorgesehen ist, durch welchen das durch die Treibladung erzeugte Gas den Treibspiegel beaufschlagt.
30. Treibladungskartusche oder KanüIen-/Treibladungskartuscheneinheit nach Aspekt 28 oder 29, wobei das Co-Volumen (81) des Ringraums durch ein an der rückwärtigen Stirnseite des Ringraums eingreifendes ringförmiges Element einstellbar ist.
31. Treibladungskartusche oder Kanülen-/Treibladungskartuscheneinheit nach einem der Aspekte 17 bis 30, wobei die Anzündvorrichtung (23) elektrisch ansteuerbar ist und dass zwei Anschlusskontakte (27a, 27b) an der rückwärtigen Stirnseite des jeweiligen Gehäuses so herausgeführt sind, dass deren Kontaktanschlussflächen innerhalb zweier gedachter konzentrischer Ringbereiche oder innerhalb eines gedachten zentrischen Kreises und eines gedachten konzentrischen Ringbereichs liegen.
32. Anzündeinrichtung für eine Treibladungskartusche oder Kanülen-/Treibladungskartuscheneinheit, insbesondere nach einem der Aspekte 17 bis 31,
   a) wobei zwei Anschlusskontakte (27a, 27b) der Anzündvorrichtung (23) der Treibladungskartusche (7) oder der Kanülen-/Treibladungskartuscheneinheit (65) an der rückwärtigen Stirnseite des jeweiligen Gehäuses so herausgeführt sind, dass deren Kontaktanschlussflächen innerhalb zweier gedachter konzentrischer Ringbereiche oder innerhalb eines gedachten zentrischen Kreises und eines gedachten konzentrischen Ringbereichs liegen, und
      wobei
   b) die Anzündeinrichtung (31) zwei ringförmige, schneidenartig ausgebildete Kontakte (29a, 29b) oder einen zentrischen Kontakt und einen ringförmigen, schneidenartig ausgebildeten Kontakt aufweist und
   c) die Treibladungskartusche (7) oder die Kanülen-/Treibladungskartuscheneinheit (65) derart mit der Anzündeinrichtung (31) koppelbar ist, dass die Kontakte (29a, 29b) der Anzündeinrichtung (31) die Anschlusskontakte (27a, 27b) der Treibladungskartusche (7) oder der Kanülen-/Treibladungskartuscheneinheit (65) beaufschlagten.
33. Anzündeinrichtung nach Aspekt 32, wobei in einem Gehäuse (3b) eine gegen die Rückstellkraft eines federnden Elements (45) verschiebbare Batterie (43) angeordnet ist und dass elektrische Verbindungsleitungen vorgesehen sind, mittels welcher die Batteriepole (47a, 47b) bei einem Verschieben der Batterie (43) um wenigstens einen vorbestimmten Weg mit den Kontakten (29a, 29b) der Anzündeinrichtung verbindbar sind.
34. Anzündeinrichtung nach Aspekt 33, wobei sich die Batterie (43) mit einem Batteriepol (47b) gegen eine elektrisch leitende flexible Membran (49) abstützt, die Bestandteil der betreffenden elektrischen Verbindungsleitung ist, wobei die Membran (49) bei Beaufschlagung mit einer Druckkraft das Verschieben der Batterie (43) um einen ausreichenden Weg ermöglicht.

## Patentansprüche

1. Nadellose Injektionsvorrichtung
a) mit einer in ein Gehäuse (3) einsetzbaren Kanüle (5), welche mit einem vorbestimmten zu injizierenden Wirkstoff (11) befüllbar ist und welche einen in einem Kanülengehäuse verschiebbaren Kolben (9) für das Auspressen des Wirkstoffs (11) aus einer im Kanülengehäuse vorgesehenen Austrittsöffnung (53) aufweist,
b) mit einer in das Gehäuse (3) einsetzbaren Treibladungskartusche (7), welche ein Kartuschengehäuse mit einer darin vorgesehenen Treibladung (19) und eine Anzündvorrichtung (23) für das Anzünden der Treibladung umfasst, und
c) mit einem in einem Gehäuseteil (3a) der Injektionsvorrichtung (1) oder im Kanülengehäuse der Kanüle (5) oder im Kartuschengehäuse verschiebbaren Treibspiegel (15), welcher nach dem Anzünden der Treibladung (19) durch den entstehenden Gasdruck aus einer Ausgangsposition in eine Endposition bewegbar ist und dessen Außenumfang gegenüber dem Innenumfang des jeweiligen Gehäuses auch während der gesamten Verschiebebewegung abdichtend ausgebildet ist, wobei der Treibspiegel (15) den im Kanülengehäuse verschiebbaren Kolben (9) beaufschlagt,
**dadurch gekennzeichnet**,
d) dass der Treibspiegel (15) im jeweiligen Gehäuse in seiner Ausgangsposition rastend gehalten ist.

2. Injektionsvorrichtung nach Anspruch 1, **dadurch gekennzeichnet, dass** im jeweiligen Gehäuse Mittel zur rastenden Halterung des Treibspiegels (15) in seiner Endposition vorgesehen sind.

3. Injektionsvorrichtung nach Anspruch 1 oder 2, **dadurch gekennzeichnet, dass** der Treibspiegel (15) auf seiner mit dem Gasdruck beaufschlagbaren Stirnseite im äußeren Bereich eine ringförmige Ausnehmung aufweist, die so beschaffen ist, dass die zwischen dem Außenumfang des Treibspiegels (15) und der ringförmigen Ausnehmung ausgebildete lidartige Wandung (63) bei Beaufschlagung mit dem Gasdruck unter vorzugsweise elastischer Verformung gegen die Innenwandung des jeweiligen Gehäuses gepresst wird, um eine Dichtwirkung zu erzeugen.

4. Injektionsvorrichtung nach einem der vorhergehenden Ansprüche, **dadurch gekennzeichnet, dass** der Treibspiegel (15) in einem Gehäuseteil (3a) der Injektionsvorrichtung (1) vorgesehen ist und dass die Treibladungskartusche (7) als separates Teil ausgebildet und in das Gehäuseteil (3a) der Injektionsvorrichtung (1) einsetzbar ist.

5. Injektionsvorrichtung nach Anspruch 4, **dadurch gekennzeichnet, dass** der Treibspiegel (15) vom Gasdruck gegen die Rückstellkraft eines federnden Elements (71) bewegbar ist.

6. Injektionsvorrichtung nach Anspruch 4 oder 5, **dadurch gekennzeichnet, dass** das Gehäuseteil (3a) als kartuschenartiges Austauschteil ausgebildet ist.

7. Injektionsvorrichtung nach einem der Ansprüche 1 bis 3, **dadurch gekennzeichnet, dass** der Treibspiegel (15) im Kanülengehäuse vorgesehen ist und dass die Treibladungskartusche (7) als separates Teil ausgebildet und in das Kanülengehäuse einsetzbar ist.

8. Injektionsvorrichtung nach einem der Ansprüche 1 bis 3, **dadurch gekennzeichnet, dass** der Treibspiegel (15) im Kanülengehäuse vorgesehen ist und dass die Treibladungskartusche' (7) als separates Teil ausgebildet und an der rückwärtigen Seite der Kanüle (5) in das Gehäuse (3) der Injektionsvorrichtung (1) einsetzbar ist.

9. Injektionsvorrichtung nach einem der Ansprüche 1 bis 3, **dadurch gekennzeichnet, dass** die Kanüle (5) und die Treibladungskartusche (7) als Kanülen-/Treibladungskartuscheneinheit (65) ausgebildet sind, wobei der Kolben (9) und der Treibspiegel (15) im gemeinsamen Kanülen-/Kartuschengehäuse vorgesehen sind.

10. Injektionsvorrichtung nach einem der Ansprüche 7 bis 9, **dadurch gekennzeichnet, dass** der Treibspiegel (15) einstückig mit dem Kolben (9) ausgebildet oder kraftschlüssig mit diesem gekoppelt ist.

11. Injektionsvorrichtung nach Anspruch 10, **dadurch gekennzeichnet, dass** der axiale Abstand zwischen den Dichtungsmitteln des Kolbens (9) und den Dichtungsmitteln des Treibspiegels (15) größer ist als der Bewegungsweg des Treibspiegels (15) zwischen seiner Ausgangsposition und seiner Endposition.

12. Injektionsvorrichtung nach einem der Ansprüche 1 bis 3, **dadurch gekennzeichnet, dass** die Kanüle (5) und die Treibladungskartusche (7) als separate Teile ausgebildet sind, wobei der Treibspiegel (15) im Kartuschengehäuse vorgesehen ist und vorzugsweise nach dem Einsetzen von Kanüle (5) und Treibladungskartusche (7) in die Injektionsvorrichtung (1) mit dem Kolben (9) kraftschlüssig oder formschlüssig gekoppelt ist.

13. Injektionsvorrichtung nach einem der vorhergehenden Ansprüche, **dadurch gekennzeichnet, dass** die Kanüle (5) mit einem rückwärtigen Bereich in das Gehäuse der Injektionsvorrichtung (1) oder das Gehäuseteil (3a) der Injektionsvorrichtung, in welchem der Treibspiegel (15) vorgesehen ist, einschraubbar oder mittels eines Bajonettverschlusses einsetzbar ist.

14. Injektionsvorrichtung nach einem der vorhergehenden Ansprüche, **dadurch gekennzeichnet, dass** die Kanüle (5) an im Wesentlichen dem gesamten Außenumfang durch das Gehäuse (3) der Injektionsvorrichtung (1) abgestützt ist.

15. Injektionsvorrichtung nach Anspruch 14, **dadurch gekennzeichnet, dass** die Kanüle (5) vorzugsweise in ihrem rückwärtigen Bereich eine Anlagefläche auf weist, die mit einer im Wesentlichen komplementär ausgebildeten Abstützfläche des Gehäuses (3) bei einer Beaufschlagung des Kolbens (9) der Kanüle (5) zum Auspressen des Wirkstoffs (11) für das Abstützen der Kanüle (5) in axialer Richtung zusammenwirkt.

16. Treibladungskartusche für eine nadellose Injektionsvorrichtung, insbesondere nach einem der vorhergehenden Anspruche,
a) mit einem Kartuschengehäuse, einer darin vorgesehenen aktivierbaren Treibladung (19) und einer Anzündvorrichtung (23) für das Aktivieren der Treibladung und
b) mit einem im Kartuschengehäuse angeordneten, gegenüber der Gehäuseinnenwandung abdichtenden Treibspiegel (15),
c) welcher durch den nach dem Anzünden der Treibladung (19) entstehenden Gasdruck zum Antreiben des Kolbens (9) der den zu injizierenden Wirkstoff enthaltenden Kanüle (5) aus einer Ausgangsposition in eine Endposition bewegbar ist und dessen Außenumfang gegenüber dem Innenumfang des Kartuschengehäuses auch während der gesamten Verschiebebewegung abdichtend ausgebildet ist,
**dadurch gekennzeichnet**,
d) dass der Treibspiegel (15) im Kartuschengehäuse in seiner Ausgangsposition rastend gehalten ist.

17. Treibladungskartusche nach Anspruch 16, **dadurch gekennzeichnet, dass** an der Innenwandung des Kartuschengehäuses Rastvorsprünge vorgesehen sind, welche mit den Umfangskanten des Treibspiegels (15) oder einer oder mehrere in seiner Umfangsfläche vorgesehenen Rastausnehmungen zusammenwirken.

18. Treibladungskartusche nach Anspruch 16 oder 17, **dadurch gekennzeichnet, dass** im Kartuschengehäuse Mittel zur rastenden Halterung des Treibspiegels (15) in seiner Endposition vorgesehen sind, wobei vorzugsweise an der Innenwandung des Kartuschengehäuses entsprechende Rastvorsprünge vorgesehen sind, welche mit den Umfangskanten des Treibspiegels (15) oder einer oder mehrere in seiner Umfangsfläche vorgesehenen Rastausnehmungen zusammenwirken.

19. Treibladungskartusche nach einem der Ansprüche 17 bis 18, **dadurch gekennzeichnet, dass** der Treibspiegel (15) auf seiner mit dem Gasdruck beaufschlagbaren Stirnseite im äußeren Bereich eine ringförmige Ausnehmung aufweist, die so beschaffen ist, dass die zwischen dem Außenumfang des Treibspiegels und der ringförmigen Ausnehmung ausgebildete lidartige Wandung (63) bei Beaufschlagung mit dem Gasdruck unter vorzugsweise elastischer Verformung gegen die Innenwandung des Kartuschengehäuses gepresst wird, um eine Dichtwirkung zu erzeugen.

20. Kanülen-/Treibladungskartuscheneinheit für eine nadellose Injektionsvorrichtung, insbesondere nach einem der Ansprüchen 1 bis 15,
a) mit einem Kanülen-/Kartuschengehäuse, einer darin vorgesehenen aktivierbaren Treibladung (19) und einer Anzündvorrichtung (23) für das Aktivieren der Treibladung,
b) mit einem im Kanülen-/Kartuschengehäuse abdichtend verschiebbaren Kolben (9) für das Auspressen des Wirkstoffs (11) aus einer im Kanülengehäuse vorgesehenen Austrittsöffnung (53) und
c) mit einem im Kanülen-/Kartuschengehäuse angeordneten, gegenüber der Gehäuseinnenwandung abdichtenden Treibspiegel (15), welcher durch den nach dem Anzünden der Treibladung (19) entstehenden Gasdruck zum Antreiben des Kolbens (9) aus einer Ausgangsposition in eine Endposition bewegbar ist und dessen Außenumfang gegenüber dem Innenumfang des Kanülen-/Kartuschengehäuses auch während der gesamten Verschiebebewegung abdichtend ausgebildet ist,
**dadurch gekennzeichnet**,
d) dass der Treibspiegel (15) im Kanülen-/Kartuschengehäuse in seiner Ausgangsposition rastend gehalten ist.

21. Kanülen-/Treibladungskartuscheneinheit nach Anspruch 20, **dadurch gekennzeichnet, dass** an der Innenwandung des Kanülen-/Kartuschengehäuses Rastvorsprünge vorgesehen sind, welche mit den Umfangskanten des Treibspiegels (15) oder einer oder mehrere in seiner Umfangsfläche vorgesehenen Rastausnehmungen zusammenwirken.

22. Kanülen-/Treibladungskartuscheneinheit nach Anspruch 20 oder 21, **dadurch gekennzeichnet, dass** im Kanülen-/Kartuschengehäuse ein Anschlag vorgesehen ist, der die Endposition des Treibspiegels (15) definiert.

23. Kanülen-/Treibladungskartuscheneinheit nach einem der Ansprüche 20 bis 22, **dadurch gekennzeichnet, dass** der Treibspiegel (15) auf seiner mit dem Gasdruck beaufschlagbaren Stirnseite im äußeren Bereich eine ringförmige Ausnehmung aufweist, die so beschaffen ist, dass die zwischen dem Außenumfang des Treibspiegels und der ringförmigen Ausnehmung ausgebildete lidartige Wandung (63) bei Beaufschlagung mit dem Gasdruck unter vorzugsweise elastischer Verformung gegen die Innenwandung des Kanülen-/Kartuschengehäuses gepresst wird, um eine Dichtwirkung zu erzeugen.

24. Kanülen-/Treibladungskartuscheneinheit nach einem der Ansprüche 20 bis 23, **dadurch gekennzeichnet, dass** der Treibspiegel (15) einstückig mit dem Kolben (9) ausgebildet oder kraftschlüssig mit diesem gekoppelt ist.

25. Kanülen-/Treibladungskartuscheneinheit nach Anspruch 24, **dadurch gekennzeichnet, dass** der axiale Abstand zwischen den Dichtungsmitteln des Kolbens (9) und den Dichtungsmitteln des Treibspiegels (15) größer ist als der Bewegungsweg des Treibspiegels (15) zwischen seiner Ausgangsposition und seiner Endposition.

26. Treibladungskartusche nach einem der Ansprüche 16 bis 19 oder Kanülen-/Treibladungskartuscheneinheit nach einem der Ansprüche 20 bis 25, **dadurch gekennzeichnet, dass** das jeweilige Gehäuse ein Co-Volumen (81) aufweist, welches mit dem Volumen des Gehäuses, in welchem das Gas erzeugbar ist, in der Ausgangsposition des Treibspiegels (15) verbunden oder durch das Bewegen des Treibspiegels (15) aus der Ausgangsposition heraus verbindbar ist.

27. Treibladungskartusche oder Kanülen-/Treibladungskartuscheneinheit nach Anspruch 26, **dadurch gekennzeichnet, dass** das Co-Volumen (81) als Ringraum ausgebildet ist, der sich um das Volumen erstreckt, in welchem das Gas erzeugbar ist.

28. Treibladungskartusche oder Kanülen-/Treibladungskartuscheneinheit nach Anspruch 27, **dadurch gekennzeichnet, dass** der Ringraum durch eine stirnseitige Wandung (89) begrenzt ist, in welcher ein oder mehrere Durchbrüche (91) vorgesehen sind, die in der Ausgangsposition durch den Treibspiegel (15) im Wesentlichen abgedichtet sind, dass in der stirnseitigen Wandung (89) wenigstens ein Durchbruch vorgesehen ist, durch welchen das durch die Treibladung erzeugte Gas den Treibspiegel beaufschlagt.

29. Treibladungskartusche oder Kanülen-/Treibladungskartuscheneinheit nach Anspruch 27 oder 28, **dadurch gekennzeichnet, dass** das Co-Volumen (81) des Ringraums durch ein an der rückwärtigen Stirnseite des Ringraums eingreifendes ringförmiges Element einstellbar ist.

30. Treibladungskartusche oder Kanülen-/Treibladungskartuscheneinheit nach einem der Ansprüche 16 bis 29, **dadurch gekennzeichnet, dass** die Anzündvorrichtung (23) elektrisch ansteuerbar ist und dass zwei Anschlusskontakte (27a, 27b) an der rückwärtigen Stirnseite des jeweiligen Gehäuses so herausgeführt sind, dass deren Kontaktanschlussflächen innerhalb zweier gedachter konzentrischer Ringbereiche oder innerhalb eines gedachten zentrischen Kreises und eines gedachten konzentrischen Ringbereichs liegen.

## Claims

1. Needleless injection device
a) having a cannula (5) which can be inserted in a housing (3) and can be filled with a predetermined active substance (11) to be injected and which has a piston (9), which is displaceable in a cannula housing, for forcing said active substance (11) out of an exit aperture (53) provided in said cannula housing;
b) having a propellant-charge cartridge (7) which can be inserted in the housing (3) and comprises a cartridge housing having a propellant charge (19) provided therein and an igniting device (23) for igniting the propellant charge; and
c) having a propellant base (15) which is displaceable in a part (3a) of the housing of the injection device (1) or in the housing of the cannula (5) or in the cartridge housing and can be moved, after the ignition of the propellant charge (19), out of an initial position and into an end position by the gas pressure produced, and whose outer periphery is constructed so as to effect sealing-off, even throughout the displacing movement, in relation to the inner periphery of the respective housing, said propellant base (15) acting upon the piston (9) which is displaceable in the cannula housing;
**characterised in that**
d) the propellant base (15) is held in its initial position in the respective housing in a latching manner.

2. Injection device according to Claim 1, **characterised in that** means for holding the propellant base (15) in its end position in a latching manner are provided in the respective housing.

3. Injection device according to Claim 1 or 2, **characterised in that**, on its end face in the outer region, which end face can be acted upon by the gas pressure, the propellant base (15) has an annular clearance which is constituted in such a way that the lid-like wall (63) constructed between the outer periphery of the propellant base (15) and said annular clearance is forced, when acted upon by the gas pressure, against the inner wall of the respective housing with preferably elastic deformation, in order to produce a sealing action.

4. Injection device according to one of the preceding claims, **characterised in that** the propellant base (15) is provided in a part (3a) of the housing of the injection device (1) and that the propellant-charge cartridge (7) is constructed as a separate part and can be inserted in said part (3a) of the housing of the injection device (1).

5. Injection device according to Claim 4, **characterised in that** the propellant base (15) can be moved by the gas pressure against the restoring force of a resilient element (71).

6. Injection device according to Claim 4 or 5, **characterised in that** the part (3a) of the housing is constructed as a cartridge-like interchangeable part.

7. Injection device according to one of Claims 1 to 3, **characterised in that** the propellant base (15) is provided in the cannula housing, and that the propellant-charge cartridge (7) is constructed as a separate part and can be inserted in said cannula housing.

8. Injection device according to one of Claims 1 to 3, **characterised in that** the propellant base (15) is provided in the cannula housing, and that the propellant-charge cartridge (7) is constructed as a separate part and can be inserted in the housing (3) of the injection device (1) on the rearward side of the cannula (5).

9. Injection device according to one of Claims 1 to 3, **characterised in that** the cannula (5) and the propellant-charge cartridge (7) are constructed as a cannula/propellant-charge-cartridge unit (65), the piston (9) and the propellant base (15) being provided in the combined cannula/cartridge housing.

10. Injection device according to one of Claims 7 to 9, **characterised in that** the propellant base (15) is constructed in one piece with the piston (9) or is coupled to said piston in a force-locking manner.

11. Injection device according to Claim 10, **characterised in that** the axial distance between the sealing means of the piston (9) and the sealing means of the propellant base (15) is greater than the path of movement of said propellant base (15) between its initial position and its end position.

12. Injection device according to one of Claims 1 to 3, **characterised in that** the cannula (5) and the propellant-charge cartridge (7) are constructed as separate parts, the propellant base (15) being provided in the cartridge housing and being coupled to the piston (9) in a force-locking or form-locking manner, preferably after the insertion of the cannula (5) and propellant-charge cartridge (7) in the injection device (1).

13. Injection device according to one of the preceding claims, **characterised in that** the cannula (5) can, via a rearward region, be screwed into, or inserted by means of a bayonet catch in, the housing of the injection device (1) or that part (3a) of the housing of said injection device in which the propellant base (15) is provided.

14. Injection device according to one of the preceding claims, **characterised in that** the cannula (5) is supported, substantially on the entire outer periphery, by the housing (3) of the injection device (1).

15. Injection device according to Claim 14, **characterised in that** the cannula (5) has, preferably in its rearward region, a contact face which interacts with a supporting face of substantially complementary construction on the housing (3), when the piston (9) of the cannula (5) is acted upon for forcing out the active substance (11), for the purpose of supporting said cannula (5) in the axial direction.

16. Propellant-charge cartridge for a needleless injection device, particularly according to one of the preceding claims,
a) having a cartridge housing, an activatable propellant charge (19) provided therein, and an igniting device (23) for activating said propellant charge; and
b) having a propellant base (15) which is disposed in the cartridge housing and effects sealing-off in relation to the inner wall of the housing,
c) and which can be moved, by the gas pressure produced after the ignition of the propellant charge (19) for driving the piston (9) of the cannula (5) containing the substance to be injected, out of an initial position and into an end position, and whose outer periphery is constructed so as to effect sealing-off, even throughout the displacing movement, in relation to the inner periphery of the cartridge housing;
**characterised in that**
d) the propellant base (15) is held in its initial position in the cartridge housing in a latching manner.

17. Propellant-charge cartridge according to Claim 16, **characterised in that** there are provided, on the inner wall of the cartridge housing, latching projections which interact with the peripheral edges of the propellant base (15) or with one or more latching clearances provided in its peripheral face.

18. Propellant-charge cartridge according to Claim 16 or 17, **characterised in that** means for holding the propellant base (15) in its end position in a latching manner are provided in the cartridge housing, there being preferably provided on the inner wall of the cartridge housing corresponding latching projections which interact with the peripheral edges of the propellant base (15) or with one or more latching clearances provided in its peripheral face.

19. Propellant-charge cartridge according to one of Claims 17 to 18, **characterised in that**, on its end face in the outer region, which end face can be acted upon by the gas pressure, the propellant base (15) has an annular clearance which is constituted in such a way that the lid-like wall (63) constructed between the outer periphery of the propellant base and said annular clearance is forced, when acted upon by the gas pressure, against the inner wall of the cartridge housing with preferably elastic deformation, in order to produce a sealing action.

20. Cannula/propellant-charge-cartridge unit for a needleless injection device, particularly according to one of Claims 1 to 15,
a) having a cannula/cartridge housing, an activatable propellant charge (19) provided therein, and an igniting device (23) for activating said propellant charge;
b) having a piston (9), which is displaceable in a sealing-off manner in the cannula/cartridge housing, for forcing the active substance (11) out of an exit aperture (53) provided in the cannula housing; and
c) having a propellant base (15) which is disposed in the cannula/cartridge housing and effects sealing-off in relation to the inner wall of the housing and which can be moved, by the gas pressure produced after the ignition of the propellant charge (19) for driving the piston (9), out of an initial position and into an end position, and whose outer periphery is constructed so as to effect sealing-off, even throughout the displacing movement, in relation to the inner periphery of the cannula/cartridge housing,
**characterised in that**
d) the propellant base (15) is held in its initial position in the cannula/cartridge housing in a latching manner.

21. Cannula/propellant-charge-cartridge unit according to Claim 20, **characterised in that** there are provided, on the inner wall of the cannula/cartridge housing, latching projections which interact with the peripheral edges of the propellant base (15) or with one or more latching clearances provided in its peripheral face.

22. Cannula/propellant-charge-cartridge unit according to Claim 20 or 21, **characterised in that** a stop, which defines the end position of the propellant base (15), is provided in the cannula/cartridge housing.

23. Cannula/propellant-charge-cartridge unit according to one of Claims 20 to 22, **characterised in that**, on its end face in the outer region, which end face can be acted upon by the gas pressure, the propellant base (15) has an annular clearance which is constituted in such a way that the lid-like wall (63) constructed between the outer periphery of the propellant base (15) and said annular clearance is forced, when acted upon by the gas pressure, against the inner wall of the cannula/cartridge housing with preferably elastic deformation, in order to produce a sealing action.

24. Cannula/propellant-charge-cartridge unit according to one of Claims 20 to 23, **characterised in that** the propellant base (15) is constructed in one piece with the piston (9) or is coupled to said piston in a force-locking manner.

25. Cannula/propellant-charge-cartridge unit according to Claim 24, **characterised in that** the axial distance between the sealing means of the piston (9) and the sealing means of the propellant base (15) is greater than the path of movement of said propellant base (15) between its initial position and its end position.

26. Propellant-charge cartridge according to one of Claims 16 to 19 or cannula/propellant-charge-cartridge unit according to one of Claims 20 to 25, **characterised in that** the respective housing has a co-volume (81) which is connected, when the propellant base (15) is in the initial position, or which can be connected as a result of the movement of said propellant base (15) out of the initial position, to that volume of the housing in which the gas can be generated.

27. Propellant-charge cartridge or cannula/propellant-charge-cartridge unit according to Claim 26, **characterised in that** the co-volume (81) is constructed as an annular space which extends around the volume in which the gas can be generated.

28. Propellant-charge cartridge or cannula/propellant-charge-cartridge unit according to Claim 27, **characterised in that** the annular space is bounded by an end-face wall (89) in which there are provided one or more openings (91) which, in the initial position, are substantially sealed off by the propellant base (15), and that there is provided, in said end-face wall (89), at least one opening through which the gas generated by the propellant charge acts upon the propellant base.

29. Propellant-charge cartridge or cannula/propellant-charge-cartridge unit according to Claim 27 or 28, **characterised in that** the co-volume (81) of the annular space can be adjusted by means of an annular element acting on the rearward end face of said annular space.

30. Propellant-charge cartridge or cannula/propellant-charge-cartridge unit according to one of Claims 16 to 29, **characterised in that** the igniting device (23) is electrically actuatable, and that two connecting contacts (27a, 27b) are led through at the rearward end face of the respective housing in such a way that their contact-connecting faces lie within two imaginary concentric annular regions or within an imaginary central circle and an imaginary concentric annular region.

## Revendications

1. Dispositif d'injection sans aiguille
a) avec une canule (5) pouvant être insérée dans un boîtier (3), qui peut être remplie d'une substance (11) prédéterminée à injecter, et qui présente un piston (9) mobile dans un boîtier de canule pour pressurer la substance (11) provenant d'un orifice d'évacuation (53) prévu dans le boîtier de canule,
b) comportant une cartouche de charge propulsive (7) pouvant être insérée dans le boîtier (3), qui comporte un boîtier de cartouche avec une charge propulsive (19) prévue dans celui-ci et un dispositif d'allumage (23) pour activer la charge propulsive,
c) comportant un miroir propulseur (15) pouvant se déplacer dans un élément de boîtier (3a) du dispositif d'injection (1) ou dans le boîtier de canule de la canule (5) ou dans le boîtier de cartouche, lequel, après l'allumage de la charge propulsive (19) sous l'effet de la pression du gaz apparaissant, peut se déplacer d'une position initiale à une position finale, et dont le pourtour extérieur est configuré pour assurer l'étanchéité également pendant tout le mouvement de déplacement par rapport au pourtour intérieur du boîtier respectif, moyennant quoi le miroir propulseur (15) sollicite le piston (9) mobile dans le boîtier de canule,
**caractérisé en ce que**
d) le miroir propulseur (15) est maintenu par encliquetage dans le boîtier respectif dans sa position initiale.

2. Dispositif d'injection selon la revendication 1, **caractérisé en ce que**, dans le boîtier respectif, sont prévus des moyens permettant de maintenir par encliquetage le miroir propulseur (15) dans sa position finale.

3. Dispositif d'injection selon l'une quelconque des revendications 1 ou 2, **caractérisé en ce que** le miroir propulseur (15), sur son côté frontal pouvant être sollicité par la pression gazeuse, présente, dans la zone extérieure, un évidement annulaire, qui est configuré de telle sorte que la paroi de type recouvrement (63) formée entre le pourtour extérieur du miroir propulseur (15) et l'évidement annulaire est comprimée lors de la sollicitation avec la pression gazeuse de préférence avec une déformation élastique contre la paroi intérieure du boîtier respectif, afin de garantir un effet d'étanchéité.

4. Dispositif d'injection selon l'une quelconque des revendications précédentes, **caractérisé en ce que** le miroir propulseur (15) est prévu dans un élément de boîtier (3a) du dispositif d'injection (1), et **en ce que** la cartouche de charge propulsive (7) est configurée comme un élément séparé et peut être insérée dans l'élément de boîtier (3a) du dispositif d'injection (1).

5. Dispositif d'injection selon la revendication 4, **caractérisé en ce que** le miroir propulseur (15) peut être déplacé sous l'effet de la pression gazeuse à l'encontre de la force de rappel d'un élément de ressort (71).

6. Dispositif d'injection selon l'une quelconque des revendications 4 ou 5, **caractérisé en ce que** l'élément de boîtier (3a) est formé par un élément interchangeable de type cartouche.

7. Dispositif d'injection selon l'une quelconque des revendications 1 à 3, **caractérisé en ce que** le miroir propulseur (15) est prévu dans le boîtier de canule, et **en ce que** la cartouche de charge propulsive (7) est configurée comme un élément séparé et peut être insérée dans le boîtier de cartouche.

8. Dispositif d'injection selon l'une quelconque des revendications 1 à 3, **caractérisé en ce que** le miroir propulseur (15) est prévu dans le boîtier de canule, et **en ce que** la cartouche de charge propulsive (7) est configurée comme un élément séparé et, sur la face arrière de la canule (5), elle peut être insérée dans le boîtier (3) du dispositif d'injection (1).

9. Dispositif d'injection selon l'une quelconque des revendications 1 à 3, **caractérisé en ce que** la canule (5) et la cartouche de charge propulsive (7) sont configurées sous forme d'unité de cartouche de canule /de charge propulsive (65), dans lequel le piston (9) et le miroir propulseur (15) sont prévus dans le boîtier de canule / cartouche commun.

10. Dispositif d'injection selon l'une quelconque des revendications 7 à 9, **caractérisé en ce que** le miroir propulseur (15) est réalisé d'une seule pièce avec le piston (9) ou par conjugaison de forme avec celui-ci.

11. Dispositif d'injection selon la revendication 10, **caractérisé en ce que** la distance axiale entre les moyens d'étanchéité du piston (9) et les moyens d'étanchéité du miroir propulseur (15) est supérieure à la trajectoire de déplacement du miroir propulseur (15) entre sa position initiale et sa position finale.

12. Dispositif d'injection selon l'une quelconque des revendications 1 à 3, **caractérisé en ce que** la canule (5) et la cartouche de charge propulsive (7) sont configurées comme des éléments séparés, dans lequel le miroir propulseur (15) est prévu dans le boîtier de cartouche et, de préférence, après l'insertion de la canule (5) et de la cartouche de charge propulsive (7) dans le dispositif d'injection (1), est accouplé par conjugaison de force ou de forme avec le piston (9).

13. Dispositif d'injection selon l'une quelconque des revendications précédentes, **caractérisé en ce que** la canule (5) peut être enfoncée par vissage ou insérée à l'aide d'une fermeture à baïonnette avec une zone arrière dans le boîtier du dispositif d'injection (1) ou dans l'élément de boîtier (3a) du dispositif d'injection dans lequel est prévu le miroir propulseur (15).

14. Dispositif d'injection selon l'une quelconque des revendications précédentes, **caractérisé en ce que** la canule (5) repose sensiblement sur l'ensemble du pourtour extérieur par l'intermédiaire du boîtier (3) du dispositif d'injection (1).

15. Dispositif d'injection selon la revendication 14, **caractérisé en ce que** la canule (5) présente de préférence dans sa zone arrière une surface de positionnement, qui coopère avec une surface d'appui du boîtier (3) configurée de manière sensiblement complémentaire, ce qui permet, lors d'une sollicitation du piston (9) de la canule (5), de comprimer la substance (11) pour soutenir la canule (5) dans la direction axiale.

16. Cartouche de charge propulsive pour un dispositif d'injection sans aiguille, en particulier selon l'une quelconque des revendications précédentes :
a) comportant un boîtier de cartouche, une charge propulsive (19) prévue à l'intérieur et pouvant être activée, et un dispositif d'allumage (23) pour activer la charge propulsive, et
b) comportant un miroir propulseur (15) disposé dans le boîtier de cartouche, rendu étanche par rapport à la paroi intérieure du boîtier,
c) qui, sous l'effet de la pression gazeuse apparaissant après l'activation de la charge propulsive (19) peut être déplacée pour entraîner le piston (9) de la canule (5) contenant la substance à injecter d'une position initiale à une position finale, et dont le contour extérieur est configuré de manière à assurer l'étanchéité pendant tout le mouvement de déplacement par rapport au contour intérieur du boîtier de cartouche,
**caractérisée en ce que**
d) le miroir propulseur (15) est maintenu par encliquetage dans le boîtier de cartouche dans sa position initiale.

17. Cartouche de charge propulsive selon la revendication 16, **caractérisée en ce que**, sur la paroi interne du boîtier de cartouche sont prévues des saillies d'encliquetage, qui coopèrent avec les bords périphériques du miroir propulseur (15) ou un ou plusieurs évidements d'encliquetage prévus dans sa surface périphérique.

18. Cartouche de charge propulsive selon l'une quelconque des revendications 16 ou 17, **caractérisée en ce que**, dans le boîtier de cartouche, sont prévus des moyens pour un maintien par encliquetage du miroir propulseur (15) dans sa position finale, dans laquelle, de préférence, sont prévues des saillies d'encliquetage correspondantes sur la paroi intérieure du boîtier de cartouche, qui coopèrent avec les bords périphériques du miroir propulseur (15) ou un ou plusieurs évidements d'encliquetage prévus dans sa surface périphérique.

19. Cartouche de charge propulsive selon l'une quelconque des revendications 17 à 18, **caractérisée en ce que** le miroir propulseur (15) présente, sur son côté frontal pouvant être sollicité par la pression gazeuse, dans la zone extérieure, un évidement annulaire, qui est configuré de telle sorte que la paroi (63) de type recouvrement configurée entre le contour extérieur du miroir propulseur et l'évidement annulaire, lors de la sollicitation par la pression gazeuse, est comprimée de préférence avec une déformation élastique contre la paroi intérieure du boîtier de cartouche, afin de produire un effet d'étanchéité.

20. Unité de canule / cartouche de charge propulsive pour un dispositif permettant l'injection sans aiguille, en particulier selon l'une quelconque des revendications 1 à 15,
a) comportant un boîtier de canule / cartouche, une charge propulsive (19) prévue à l'intérieur et pouvant être activée, et un dispositif d'allumage (23) permettant d'activer la charge propulsive,
b) comportant un piston (9) mobile avec un effet d'étanchéité dans le boîtier de canule / cartouche, permettant de comprimer la substance (11) à partir d'un orifice d'évacuation (53) prévu dans le boîtier de canule, et
c) comportant un miroir propulseur (15) disposé dans le boîtier de canule / cartouche, assurant l'étanchéité par rapport à la paroi intérieure du boîtier, qui, sous l'effet de la pression gazeuse apparaissant après l'allumage de charge propulsive (19), peut se déplacer pour entraîner le piston (9) d'une position initiale à une position finale, et dont le contour extérieur est configuré pour assurer l'étanchéité également pendant tout le mouvement de déplacement par rapport au contour intérieur du boîtier de canule / cartouche
**caractérisée en ce que**
d) le miroir propulseur (15) est maintenu par encliquetage dans le boîtier de canule / de cartouche dans sa position initiale.

21. Unité de canule / cartouche selon la revendication 20, **caractérisée en ce que**, sur la paroi intérieure du boîtier de canule / cartouche, sont prévues des saillies d'encliquetage, qui coopèrent avec les bords périphériques du miroir propulseur (15) ou un ou plusieurs évidements d'encliquetage prévus dans sa surface périphérique.

22. Unité de canule / charge propulsive selon l'une quelconque des revendications 20 ou 21, **caractérisée en ce que**, dans le boîtier de canule / cartouche, il est prévu une butée qui définit la position finale du miroir propulseur (15).

23. Unité de canule / cartouche de charge propulsive selon l'une quelconque des revendications 20 à 22, **caractérisée en ce que** le miroir propulseur (15) présente, sur son côté frontal pouvant être sollicité par la pression gazeuse, dans sa zone extérieure, un évidement annulaire qui est réalisé de telle sorte que la paroi de type recouvrement (63) configurée entre le contour extérieur du miroir propulseur et l'évidement annulaire, lors de la sollicitation avec la pression gazeuse, est comprimée de préférence avec une déformation élastique contre la paroi intérieure du boîtier de canule / cartouche, afin de produire l'effet d'étanchéité.

24. Unité de canule / cartouche de charge propulsive selon l'une quelconque des revendications 20 à 23, **caractérisée en ce que** le miroir propulseur (15) est réalisé d'un seul tenant avec le piston (9) ou est accouplé par conjugaison de forme avec celui-ci.

25. Unité de canule / cartouche de charge propulsive selon la revendication 24, **caractérisée en ce que** la distance axiale entre les moyens d'étanchéité du piston (9) et les moyens d'étanchéité du miroir propulseur (15) est supérieure à la trajectoire de déplacement du miroir propulseur (15) entre sa position initiale et sa position finale.

26. Cartouche de charge propulsive selon l'une quelconque des revendications 16 à 19 ou unité de canule /cartouche de charge propulsive selon l'une quelconque des revendications 20 à 25, **caractérisée en ce que** le boîtier respectif présente un co-volume (81), qui peut être associé au volume du boîtier dans lequel le gaz peut être produit, dans la position initiale du miroir propulseur (15), ou qui peut être relié sous l'effet du déplacement du miroir propulseur (15) à partir de la position initiale.

27. Cartouche de charge propulsive ou unité de canule / cartouche de charge propulsive selon la revendication 26, **caractérisée en ce que** le co-volume (81) est configuré sous forme d'espace annulaire, qui s'étend autour du volume dans lequel peut être produit le gaz.

28. Cartouche de charge propulsive ou unité de canule / cartouche de charge propulsive selon la revendication 27, **caractérisée en ce que** l'espace annulaire est délimité par une paroi (89) du côté frontal, dans laquelle sont prévus un ou plusieurs ajours (91) qui sont rendus sensiblement étanches dans la position initiale par le miroir propulseur (15) et, dans la paroi du côté frontal (89), est prévu au moins un ajour, par l'intermédiaire duquel le gaz produit par la charge propulsive sollicite le miroir propulseur.

29. Cartouche de charge propulsive ou unité de canule / cartouche de charge propulsive selon l'une quelconque des revendications 27 ou 28, **caractérisée en ce que** le co-volume (81) de l'espace annulaire peut être ajusté par un élément de forme annulaire mordant dans le côté frontal à l'arrière de l'espace annulaire.

30. Cartouche de charge propulsive ou unité de canule / cartouche de charge propulsive selon l'une quelconque des revendications 16 à 29, **caractérisée en ce que** le dispositif d'allumage (23) peut être commandé par voie électrique, et **en ce que** deux contacts de connexion (27a, 27b) sont réalisés de telle sorte sur le côté frontal à l'arrière du boîtier respectif que les surfaces de connexion par contact se trouvent à l'intérieur de deux zones annulaires concentriques ménagées à cet effet ou à l'intérieur d'un cercle centré ménagé à cet effet et d'une zone annulaire concentrique ménagée à cet effet.
